# EUROPEAN PATENT SPECIFICATION

(11) **EP 1 618 126 B1**
(45) Date of publication and mention of the grant of the patent: **02.02.2011**
(21) Application number: 04727194.5
(22) Date of filing: 14.04.2004
(51) Int. Cl.: C07K 14/165

(54) **SARS-RELATED PROTEINS**
SARS-VERWANDTE PROTEINE
PROTEINES ASSOCIEES AU SRAS

(30) Priority: 15.04.2003 US 462688 P; 22.05.2003 US 472416 P
(43) Date of publication of application: 25.01.2006
(73) Proprietor: Her Majesty The Queen In Right of Canada as represented by The Minister of Health, Winnipeg, Manitoba R3E 3P6 (CA); University Of Manitoba, Winnipeg, MB R3T 5V4 (CA)
(72) Inventor: JONES, Steven, Winnipeg, Manitoba R3E 3P6 (CA); FELDMANN, Heinz, Winnipeg, Manitoba R3E 3P6 (CA); BASTIEN, Nathalie, Winnipeg, Manitoba R3E 3P6 (CA); LI, Yan, Winnipeg, Manitoba R3E 3P6 (CA); PLUMMER, Frank, Winnipeg, Manitoba R3E 3P6 (CA); ENS, Werner, Winnipeg, Manitoba R3T 5V4 (CA); STANDING, Kenneth, Winnipeg, Manitoba R3T 5V4 (CA); KROKHINE, Oleg, Winnipeg, Manitoba R3T 5V4 (CA); PERREAULT, Helen, Winnipeg, Manitoba R3T 5V4 (CA); TYLER, Shaun, Winnipeg, Manitoba R3E 3P6 (CA)
(74) Representative: Jostarndt, Hans-Dieter
(86) International application number: PCT/CA2004/000558
(87) International publication number: WO 2004/092208

(56) References cited:
- DROSTEN CHRISTIAN ET AL: "Identification of a novel coronavirus in patients with severe acute respiratory syndrome." THE NEW ENGLAND JOURNAL OF MEDICINE. 15 MAY 2003, vol. 348, no. 20, 10 April 2003 (2003-04-10), pages 1967-1976, XP002305233 ISSN: 1533-4406
- KSIAZEK T G ET AL: "A novel coronavirus associated with severe acute respiratory syndrome" NEW ENGLAND JOURNAL OF MEDICINE, MASSACHUSETTS MEDICAL SOCIETY, BOSTON, MA, US, vol. 348, no. 20, 10 April 2003 (2003-04-10), pages 1953-1966, XP002276003 ISSN: 1533-4406
- POUTANEN S M ET AL: "Identification of severe acute respiratory syndrome in Canada" NEW ENGLAND JOURNAL OF MEDICINE, THE, MASSACHUSETTS MEDICAL SOCIETY, WALTHAM, MA, US, vol. 348, no. 20, 31 March 2003 (2003-03-31), pages 1995-2005, XP002255633 ISSN: 0028-4793
- KROKHIN O ET AL: "MASS SPECTROMETRIC CHARACTERIZATION OF PROTEINS FROM THE SARS VIRUS" MOLECULAR AND CELLULAR PROTEOMICS, AMERICAN SOCIETY FOR BIOCHEMISTRY AND MOLECULAR BIOLOGY, US, vol. 2, no. 5, May 2003 (2003-05), pages 346-356, XP001183239 ISSN: 1535-9476
- MARRA M A ET AL: "The genome sequence of the SARS-associated coronavirus" SCIENCE, AMERICAN ASSOCIATION FOR THE ADVANCEMENT OF SCIENCE,, US, vol. 300, no. 5624, 30 May 2003 (2003-05-30), pages 1399-1404, XP002269483 ISSN: 0036-8075

## Description

### PRIOR APPLICATION INFORMATION

This application claims priority on USSN 60/462,688, filed April 15, 2003 and USSN 60/472,416, filed May 22, 2003.

### FIELD OF INVENTION

The present invention relates to novel proteins isolated from a patient with Severe Acute Respiratory Syndrome (SARS). The invention also relates to the uses of said proteins in the therapy and diagnosis of SARS.

### BACKGROUND OF THE INVENTION

The recent clinical identification of a novel type of atypical pneumonia without a clearly defined etiology, together with epidemiological evidence of high transmissibility, have provoked the World Health Organization to issue a rare travel advisory. The new entity has been called severe acute respiratory syndrome (SARS); it apparently began in Guangdong province in China in November of 2002, and it has since spread to Hong Kong, Singapore, Vietnam, Canada, the USA, Taiwan and several European countries. The outbreak in Canada began in late February 2003 in a traveler returning from Hong Kong, whose exposure was to the index case in the Hong Kong epidemic (a physician who had cared for SARS cases in Guangdong province in the People's Republic of China). The Canadian index case died nine days after the disease onset and a 43-year-old male relative became ill 2 days after exposure and died of the adult respiratory distress syndrome 15 days after the illness began (1). Subsequently Canada has faced the largest SARS outbreak outside of Asia with at least 303 probable and suspected cases and 24 deaths, mostly in the Toronto area (2).

Samples from patients with suspect or probable SARS in Canada have been referred to the National Microbiology Laboratory (NML), Health Canada, for laboratory diagnostics. This laboratory, part of the Canadian Science Centre for Human and Animal Health, is Canada's national reference centre for infectious diseases, and houses the only Class 4 containment facilities in the country. NML has played an active role in an intensive international collaborative effort among eleven laboratories around the world that suggested a distinct coronavirus may be etiologically involved. In particular, the laboratory prepared the nucleotide samples for the first successful effort to determine the genome sequence for the coronavirus (3), a result soon confirmed by several other laboratories; see, for example, ref. (4).

Nevertheless, the genome sequence merely provides a template for the construction of the viral proteins. Thus an alternative strategy is to examine the proteins themselves, and mass spectrometry has proved to be an efficient tool for this purpose (5).

### SUMMARY OF THE INVENTION

The present inventors have isolated and sequenced novel proteins from a biological sample from a patient with Severe Acute Respiratory Syndrome (SARS).

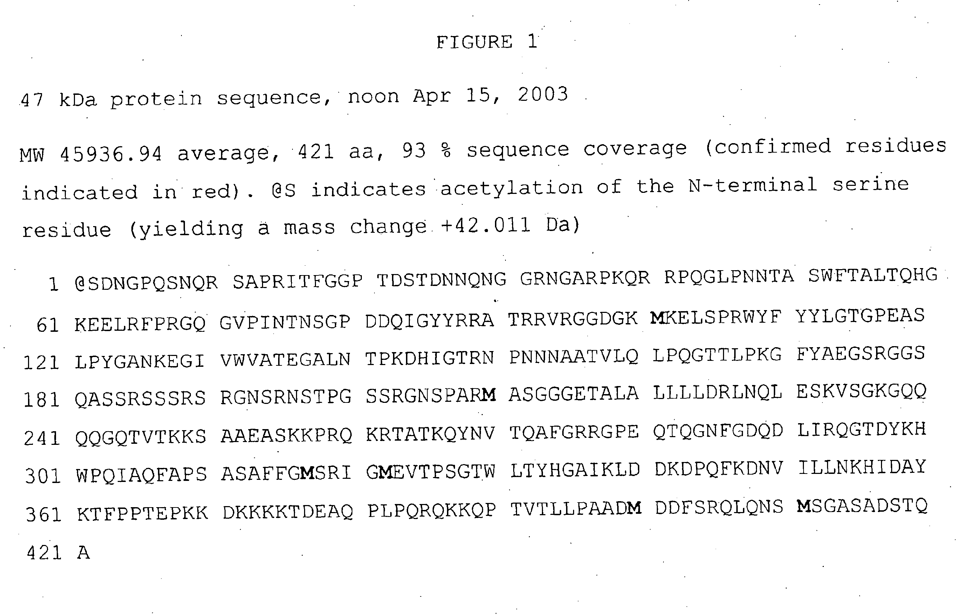

In accordance with one embodiment of the disclosure there is provided an isolated SARS-related protein from a SARS patient. In a specific embodiment, the SARS-related protein has the amino acid sequence shown in Figure 1 (SEQ ID No. 1) or 2 (SEQ ID No. 2).

This disclosure is also directed to the use of the SARS-related proteins or a fragment thereof for the preparation of a vaccine to treat SARS.

This disclosure is directed to the use of the SARS-related proteins or a fragment thereof for the preparation of antibodies, including polyclonal antibodies, monoclonal antibodies or functional fragment thereof. This disclosure also relates to the antibodies so produced.

This disclosure is also directed to vaccines prepared from said antibodies or SARS-related proteins or a fragment thereof, for the treatment or prevention of virally mediated, preferably, coronavirus mediated, diseases.

This disclosure is directed to the use of said antibodies or SARS-related proteins, or a fragment thereof, as a diagnostic to screen for the presence of SARS.

This disclosure is directed to the use of the SARS-related protein or a fragment thereof as a drug development tool.

The is yet further directed to a method of treating a condition associated with a virus, preferably a coronavirus, comprising of administering to a cell or animal in need thereof, an effective amount of agent that modulates the expression and/or activity of a SARS-related protein of the invention. In a preferred embodiment, the condition to be treated is SARS.

### BRIEF DESCRIPTION OF THE DRAWINGS

These and other features of the invention will become more apparent from the following description in which reference is made to the appended drawings wherein:
Figure 1 shows the amino acid sequence of the 47 kDa SARS-related protein (SEQ ID No. 1).

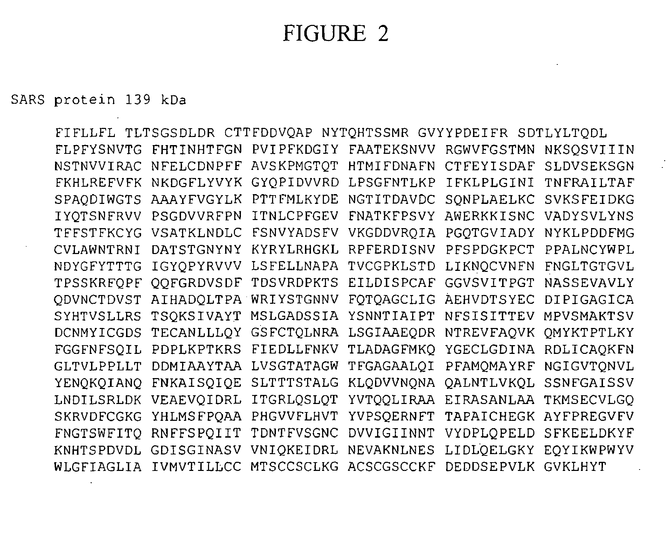

Figure 2 shows the amino acid sequence of the 139 kDa SARS-related protein (SEQ ID No. 2).

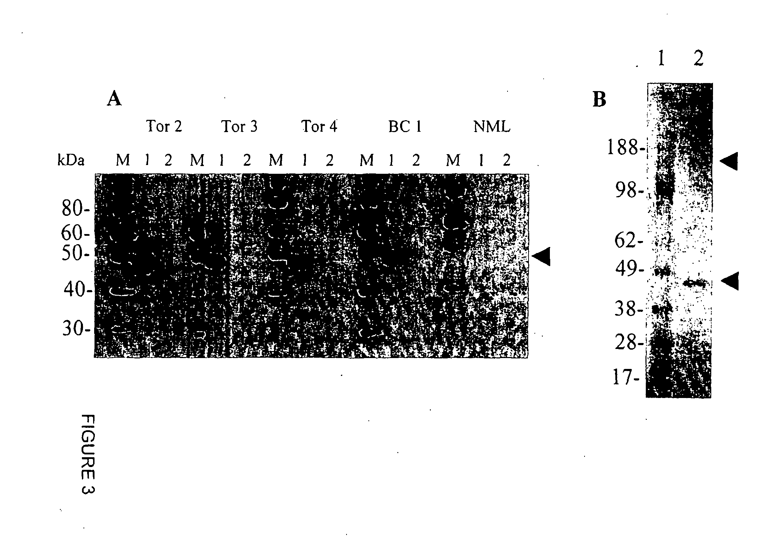

Figure 3 shows the analysis of proteins from a new corona virus associated with SARS. A) The viral particles pelleted from the supernatant of vero E6 cells exposed to samples derived from patients with SARS (lanes 1) or from mock infected cells processed in a similar fashion (lanes 2) were separated by SDS PAGE and analysed by western blot with convalescent sera from SARS patients (Tor 2, Tor 3, Tor 4, and BC 1) or a control serum from a non infected donor (NML). The sera from the patients but not the control reacted with a 44-48 kDa species present in the supernatants from the infected but not the mock treated cultures (indicated by arrow head). B) A virus sample similar to that described in A) was fractionated on a sucrose gradient. The fraction containing immunoreactive material was separated on a 4-12% bis acrylamide gradient gel and stained with colloidal Coomasie blue. A prominent band with an apparent molecular weight of 44-48 kDa was observed along with much less intensive at ∼180 kDa (indicated by arrow head). These bands were excised and used for the mass spectrometric studies described in the text.

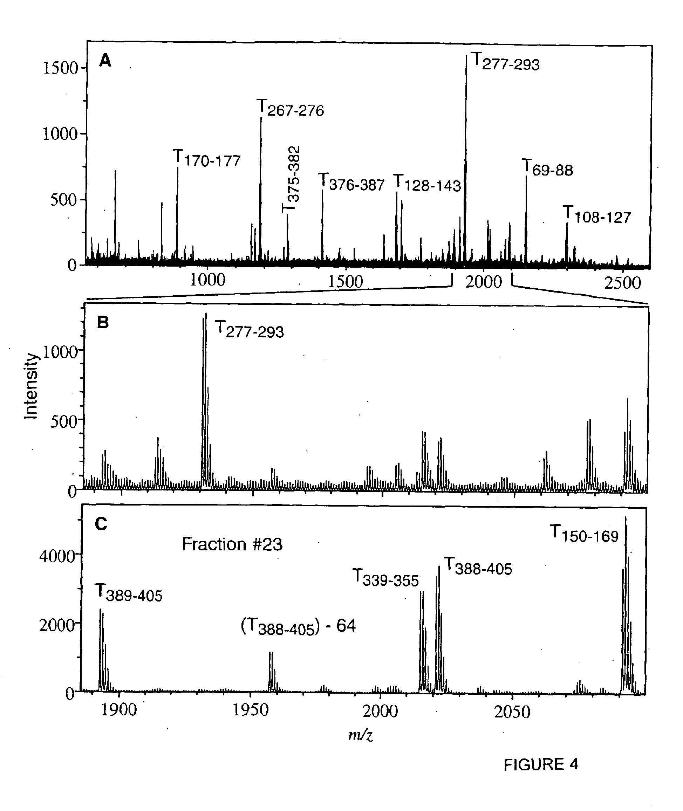

Figure 4. A) Single-MS spectrum of the peptide mixture obtained from tryptic digestion of the 47 kDa protein prior to HPLC fractionation. B) A small section of the MALDI mass spectrum of a mixture of tryptic peptides from the 47 kDa protein. C) Same section of the spectrum obtained from fraction 23 of the HPLC separation of the mixture. The peak labels indicate the residue numbers corresponding to the intact protein; in one case loss of 64 Da is indicated. The intense peak corresponding to T277-293 in the mixture is absent in fraction 23 (it elutes in fraction 21), but several weaker peaks that are present, like T389-405, are significantly enhanced by the HPLC. The improvement helps to identify them, and is essential for high mass accuracy and for subsequent MS/MS analysis. Measured and predicted masses for all the tryptic peptides can be found in Table 2; Dm is less than 10 mDa in nearly every case.

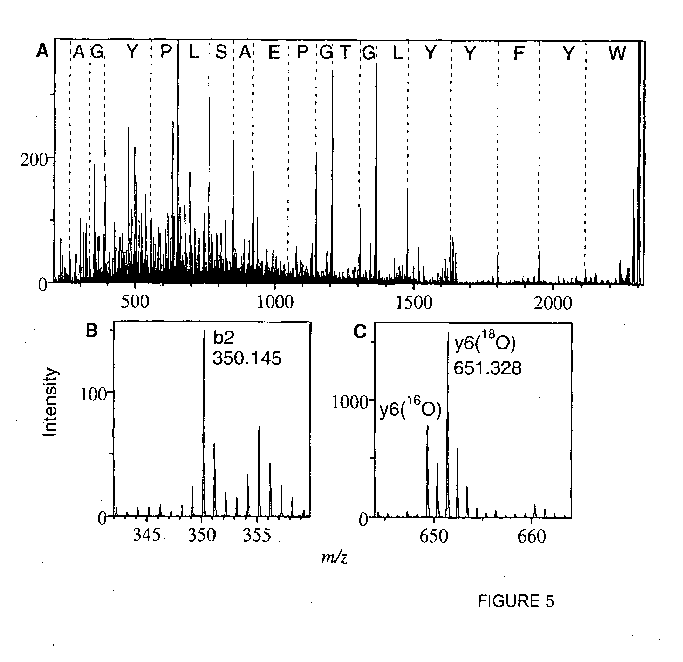

Figure 5. MS/MS spectrum of tryptic fragment of m/z 2297 after digestion in 50/50 mixture of normal and O-18 labeled water. The complete spectrum is shown in A, with the amino acid sequence indicated between the y-series fragments. An example of a b-series fragment is shown in B, and of a y-series fragment in C. The signature isotopic pattern of fragments containing the C-terminus is visible in C. The measured and predicted masses for all identified peaks are shown in Table 1.

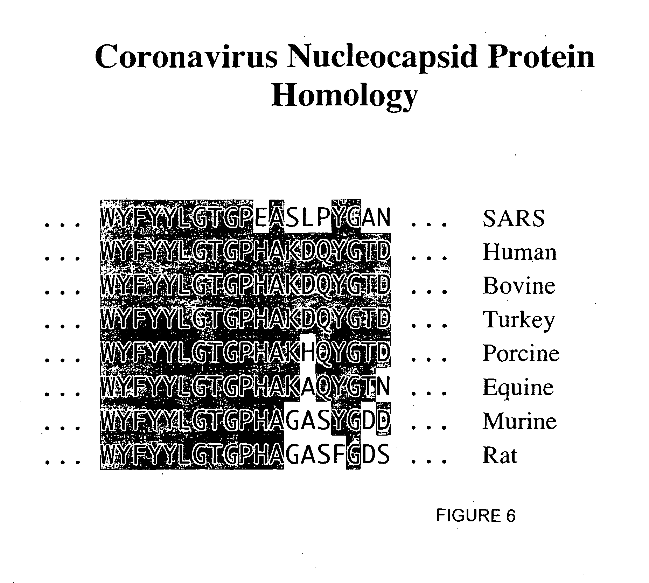

Figure 6 shows the sequence alignment of the coronovirus nucleocapsid protein.

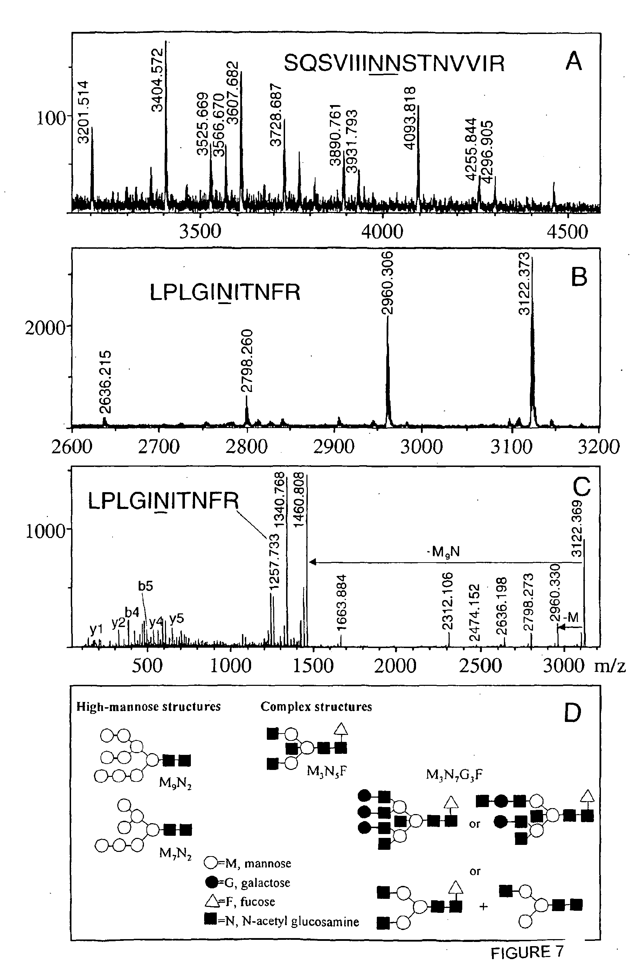

Figure 7. Direct MS (A, B) and MS/MS (C) detection of glycosylated tryptic peptides of the 139 kDa spike protein. A) MS spectrum of HPLC fraction #21 of tryptic digest of the 180 kDa band. Labeled peaks are the monoisotopic [M+H] + ions of glycosylated forms of T111-126 (see Table 3). B) MS spectrum of HPLC fraction #25 showing forms of glycosylated T222-232. C) MS/MS spectra of the 3122.373 Da peak from B. D) Suggested N-glycan structures.

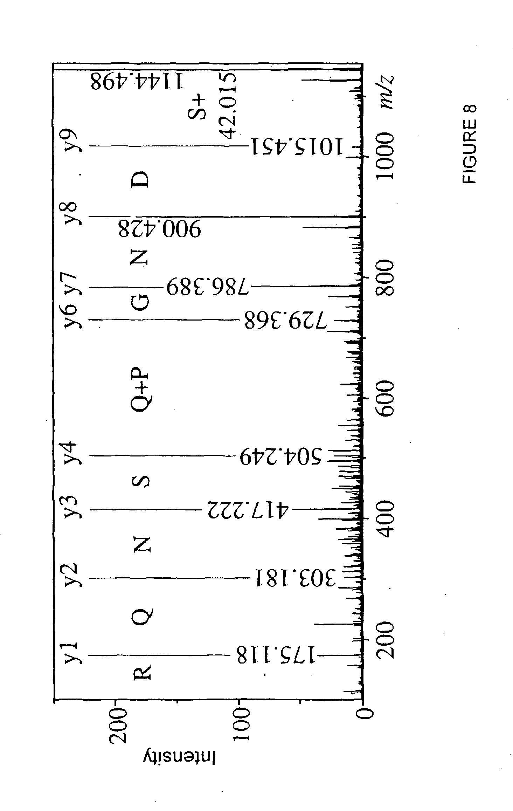

Figure 8. MS/MS spectrum of the tryptic fragment of m/z 1144 representing the N-terminal end of the 47 kDa protein. The amino acid sequence is indicated between the y-series fragments. The difference between measured and calculated masses of N-terminal Ser residue (42.015 Da) correspond to N-terminal acetylation, a post-translational modification (Dm=42.011 Da).

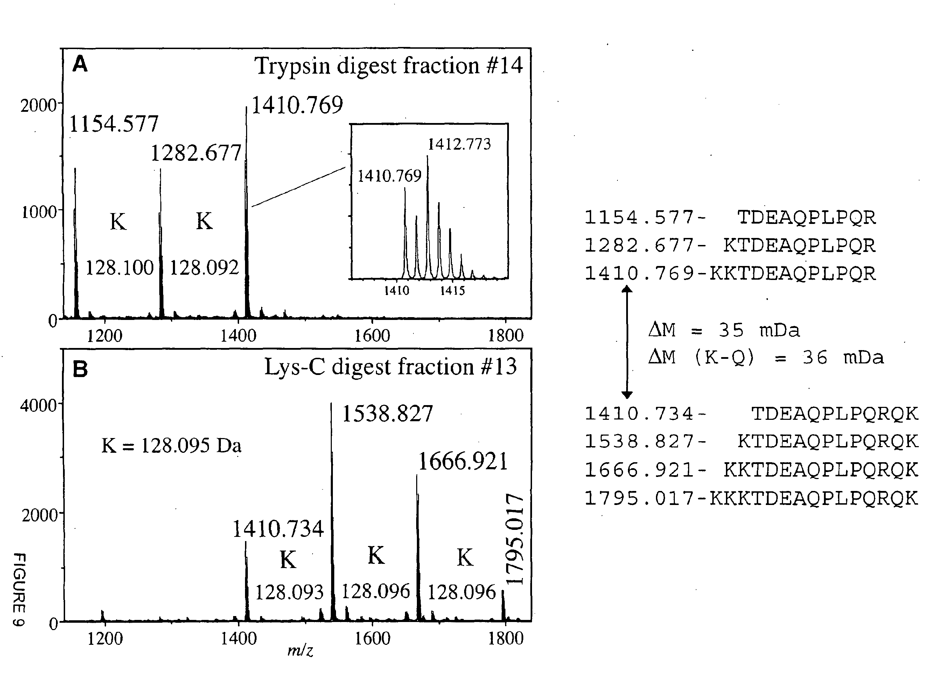

Figure 9. Comparison of (A) tryptic and (B) Lys-C digestion for the same region of the 47 kDa protein. Two additional residues (QK) are present in the Lys-C digest because it does not cleave at arginine. Both digestions were performed in the presence of a 50/50 mixture of normal and O-18 water. The signature isotopic pattern shown in the inset (A) is a characteristic of all digestion fragments except the one that contains the C-terminal of the protein. The two peaks at nominal m/z 1410 in A and B correspond to peptides containing the same residues except for a K/Q difference. The expected mass difference is 36 mDa; our measurement shows a 35 mDa difference.

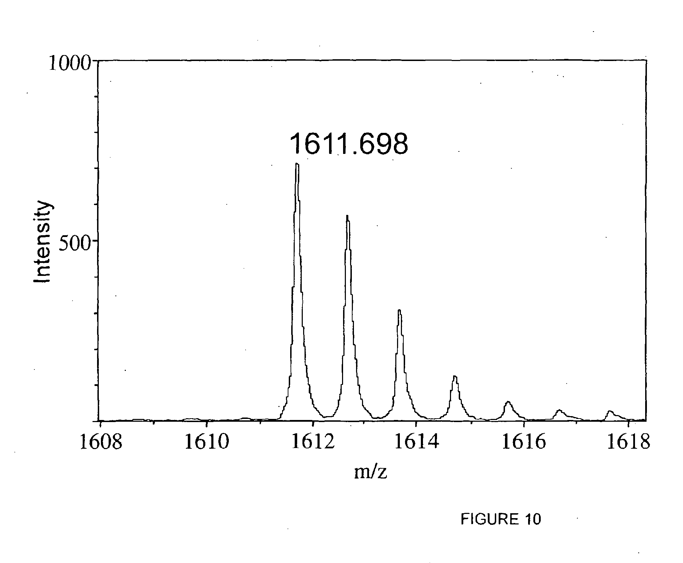

Figure 10. Mass spectrum of fraction # 12 of the 46 kDa protein tryptic digest. The digestion took place in the presence of a 50/50 mixture of normal and O-18 water. The peak shown does not exhibit the characteristic isotopic pattern for O-18 C-terminal labeling after tryptic cleavage, suggesting it is the C-terminal peptide. This was confirmed by subsequent MS/MS analysis, which reveals a sequence consistent with the C-terminus of the predicted 46 kDa protein: QLQNSM*SGASADSTQA (M* - oxidized methionine residue).

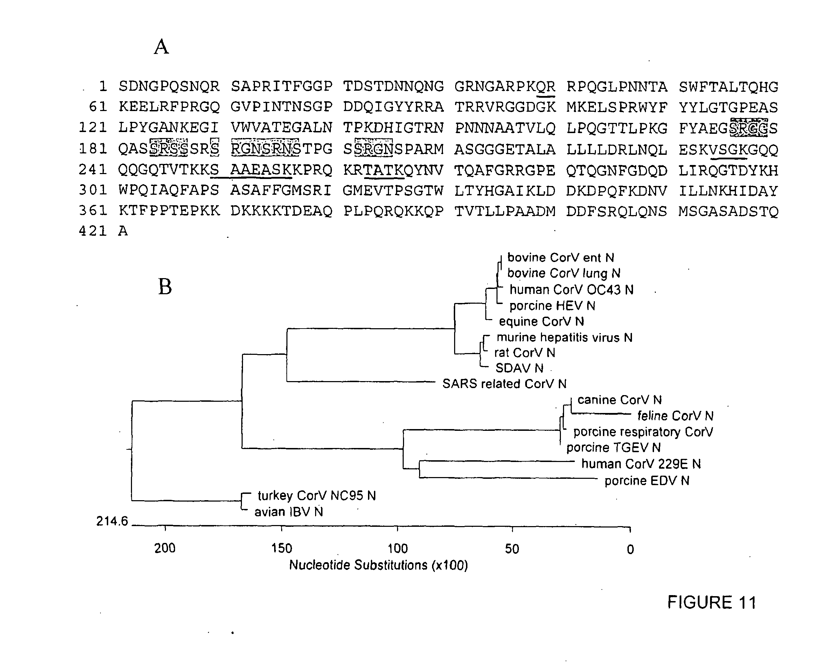

Figure 11. A) The deduced sequence of the 47-kDa SARS nucleocapsid protein. The underlined sequences were not identified by MS. The 5 SRXX motifs are indicated by red letters. B) Phylogenetic tree analysis of different coronavirus N protein sequences.

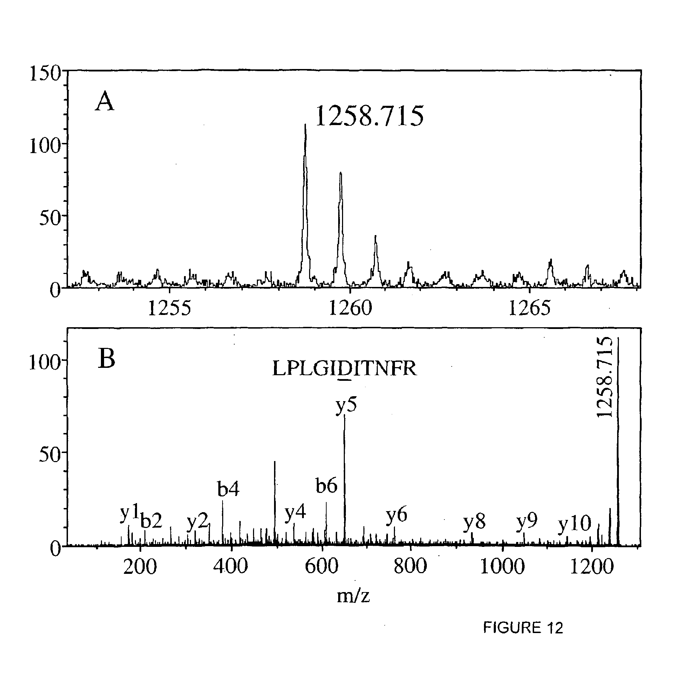

Figure 12. MS and MS/MS identification of deglycosylated peptides from the139 kDa spike protein. A) MS spectrum of HPLC fraction #26 showing deglycosylated T222-232. B) MS/MS spectrum of deglycosylated LPLGIDITNFR at 1258.715 Da (see Table 4).

### DESCRIPTION OF PREFERRED EMBODIMENT

The inventors have determined that sera from a SARS patient contained a dominant viral antigen that was identified by matrix-assisted laser desorption/ionization time-of-flight mass spectrometry (MALDI-TOFMS) as a ∼47 kDa protein species. This was found to be a novel nucleocapsid protein. A second viral protein corresponding to the predicted ∼139 kDa spike glycoprotein has also been examined by MALDI-TOFMS (42% coverage) (Krokhin et al., 2003, Mol. Cell. Proteomics 2: 346-356,). After pNGase F digestion, nine glycosylation sites in this protein were confirmed. The sugars attached to four of the sites were also identified. These results suggest that the nucleocapsid protein and the spike glycoprotein are major immunogens that may be useful for early diagnostics, or of prophylactic value in combating SARS.

### I. Novel Proteins

The disclosure relates to isolated proteins that have been isolated and sequenced from a patient with SARS. For ease of referral, the proteins are generally referred to SARS-related proteins. The term "SARS-related proteins" as used herein includes the proteins shown in Figures 1 (SEQ ID No. 1) and 2 (SEQ ID No. 2) as well as all homologs, analogs, fragments or derivatives of the SARS-related proteins.

In one embodiment, the isolated SARS-related protein has a molecular weight of approximately 47 kDa and an amino acid sequence as shown in Figure 1. Sequence analysis reveals that this protein shares 32% homology with coronavirus nucleocapsid proteins (Figure 6) although there are significant differences in the sequences. Accordingly, the present disclosure provides an isolated nucleocapsid protein comprising a sequence that is at least 35% homologous, preferably at least 40% homologous with the sequence shown in Figure 1. The inventors have further characterized the nucleocapsid protein using mass spectroscopy as described in Example 1 and the Figures and Tables.

In another embodiment, the isolated SARS-related protein has a molecular weight of 139 kDa and an amino acid sequence shown in Figure 2. Sequence analysis reveals that this protein shares homology to coronavirus spike glycoproteins and structural proteins. The inventors have further characterized the spike glycoprotein using mass spectroscopy as described in Example 1 and the Figures and Tables.

Within the context of the present invention, a SARS-related protein may include various structural forms of the primary protein which retain biological activity. For example, a SARS-related protein may be in the form of acidic or basic salts or in neutral form. In addition, individual amino acid residues may be modified by oxidation or reduction.

In addition to the full length amino acid sequence, the SARS-related proteins may also include truncations of the protein, and analogs, and homologs of the protein and truncations thereof as described herein. Truncated SARS-related proteins or fragments may comprise peptides of at least 5, preferably 10 and more preferably 15 amino acid residues of the sequence shown in Figure 1 or 2. In other embodiments, truncated SARS-related proteins or fragments may comprise peptides of at least 5, at least 6, at least 7, at least 8, preferably 10 or more preferably 15 consecutive or contiguous amino acid residues of the sequence shown in Figure 1 or 2.

The disclosure further provides polypeptides comprising at least one functional domain or at least one antigenic determinant of a SARS-related protein.

Analogs of the protein and/or truncations thereof as described herein, may include, but are not limited to an amino acid sequence containing one or more amino acid substitutions, insertions, and/or deletions. Amino acid substitutions may be of a conserved or non-conserved nature. Conserved amino acid substitutions involve replacing one or more amino acids of the proteins with amino acids of similar charge, size, and/or hydrophobicity characteristics. When only conserved substitutions are made the resulting analog should be functionally equivalent. Non-conserved substitutions involve replacing one or more amino acids of the amino acid sequence with one or more amino acids which possess dissimilar charge, size, and/or hydrophobicity characteristics.

One or more amino acid insertions may be introduced into the amino acid sequences. Amino acid insertions may consist of single amino acid residues or sequential amino acids ranging from 2 to 15 amino acids in length. For example, amino acid insertions may be used to destroy target sequences so that the protein is no longer active. This procedure may be used *in vivo* to inhibit the activity of a protein.

Deletions may consist of the removal of one or more amino acids, or discrete portions from the amino acid sequence of the SARS-related protein. The deleted amino acids may or may not be contiguous. The lower limit length of the resulting analog with a deletion mutation is about 10 amino acids, preferably 100 amino acids.

According to another embodiment, there are provided nucleic acid molecules deduced from the amino acid sequences or fragments thereof of the SARS-related proteins. As will be appreciated by one of skill in the art, the nucleic acid molecules may be inserted into any expression vector known in the art for expression in a suitable host. Furthermore, it is of note that the codons selected for the nucleic acid molecules may be selected in accordance with codon usage tables for the intended expression system host, thereby maximizing expression.

Analogs of a protein may be prepared by introducing mutations in the nucleotide sequence encoding the protein. Mutations in nucleotide sequences constructed for expression of analogs of a protein must preserve the reading frame of the coding sequences. Furthermore, the mutations will preferably not create complementary regions that could hybridize to produce secondary mRNA structures, such as loops or hairpins, which could adversely affect translation of the receptor mRNA.

Mutations may be introduced at particular loci by synthesizing oligonucleotides containing a mutant sequence, flanked by restriction sites enabling ligation to fragments of the native sequence. Following ligation, the resulting reconstructed sequence encodes an analog having the desired amino acid insertion, substitution, or deletion.

Alternatively, oligonucleotide-directed site specific mutagenesis procedures may be employed to provide an altered gene having particular codons altered according to the substitution, deletion, or insertion required. Deletion or truncation of a protein may also be constructed by utilizing convenient restriction endonuclease sites adjacent to the desired deletion. Subsequent to restriction, overhangs may be filled in, and the DNA religated. Exemplary methods of making the alterations set forth above are disclosed by Sambrook et al (Molecular Cloning: A Laboratory Manual, 2nd Ed., Cold Spring Harbor Laboratory Press, 1989).

The SARS-related proteins also include homologs of the amino acid sequence of the SARS-related protein and/or truncations thereof as described herein. Such homologs are proteins whose amino acid sequences are comprised of amino acid sequences that hybridize under stringent hybridization conditions, for example, such homologs will cross-react with antibodies raised against the SARS-related proteins or fragments thereof. Homologs of a SARS-related protein of the invention will have the same regions which are characteristic of the protein.

A homologous protein includes a protein with an amino acid sequence having at least 60%, preferably at least 70%, more preferably 80-95% identity with the amino acid sequence of the SARS-related protein.

The disclosure also contemplates isoforms of the proteins. An isoform contains the same number and kinds of amino acids as a protein, but the isoform has a different molecular structure. The isoforms are those having the same properties as a protein as described herein.

The present disclosure also includes a protein conjugated with a selected protein, or a selectable marker protein to produce fusion proteins. For example, the SARS cDNA sequence, for example, as deduced from the amino acid sequence of one of the SARS-related proteins, as discussed above is inserted into a vector that contains a nucleotide sequence encoding another peptide (e.g. GST-glutathione succinyl transferase). The fusion protein is expressed and recovered from prokaryotic (e.g. bacterial or baculovirus) or eukaryotic cells. The fusion protein can then be purified by affinity chromatography based upon the fusion vector sequence and the SARS-related protein obtained by enzymatic cleavage of the fusion protein.

The proteins (including truncations, analogs, etc.) may be prepared using recombinant DNA methods. Accordingly, nucleic acid molecules having a sequence which encodes a protein may be incorporated according to procedures known in the art into an appropriate expression vector which ensures good expression of the protein. Possible expression vectors include but are not limited to cosmids, plasmids, or modified viruses (e.g., replication defective retroviruses, adenoviruses and adeno-associated viruses), so long as the vector is compatible with the host cell used. The expression "vectors suitable for transformation of a host cell", means that the expression vectors contain a nucleic acid molecule and regulatory sequences, selected on the basis of the host cells to be used for expression, which are operatively linked to the nucleic acid molecule. "Operatively linked" is intended to mean that the nucleic acid is linked to regulatory sequences in a manner which allows expression of the nucleic acid.

The disclosure therefore contemplates a recombinant expression vector of the invention containing a nucleic acid molecule, or a fragment thereof, and the necessary regulatory sequences for the transcription and translation of the inserted protein-sequence. Suitable regulatory sequences may be derived from a variety of sources, including bacterial, fungal, or viral genes (For example, see the regulatory sequences described in Goeddel, Gene Expression Technology: Methods in Enzymology 185, Academic Press, San Diego, CA (1990). Selection of appropriate regulatory sequences is dependent on the host cell chosen, and may be readily accomplished by one of ordinary skill in the art. Examples of such regulatory sequences include: a transcriptional promoter and enhancer or RNA polymerase binding sequence, a ribosomal binding sequence, including a translation initiation signal. Additionally, depending on the host cell chosen and the vector employed, other sequences, such as an origin of replication, additional DNA restriction sites, enhancers, and sequences conferring inducibility of transcription may be incorporated into the expression vector. It will also be appreciated that the necessary regulatory sequences may be supplied by the native protein and/or its flanking regions.

The disclosure further provides a recombinant expression vector comprising a DNA nucleic acid molecule cloned into the expression vector in an antisense orientation. That is, the DNA molecule is operatively linked to a regulatory sequence in a manner which allows for expression, by transcription of the DNA molecule, of an RNA molecule which is antisense to a nucleotide sequence. Regulatory sequences operatively linked to the antisense nucleic acid can be chosen which direct the continuous expression of the antisense RNA molecule.

The recombinant expression vectors may also contain a selectable marker gene which facilitates the selection of host cells transformed or transfected with a recombinant molecule. Examples of selectable marker genes are genes encoding a protein such as G418 and hygromycin which confer resistance to certain drugs, β-galactosidase, chloramphenicol acetyltransferase, or firefly luciferase. Transcription of the selectable marker gene is monitored by changes in the concentration of the selectable marker protein such as β-galactosidase, chloramphenicol acetyltransferase, or firefly luciferase. If the selectable marker gene encodes a protein conferring antibiotic resistance such as neomycin resistance transformant cells can be selected with G418. Cells that have incorporated the selectable marker gene will survive, while the other cells die. This makes it possible to visualize and assay for expression of recombinant expression vectors and in particular to determine the effect of a mutation on expression and phenotype. It will be appreciated that selectable markers can be introduced on a separate vector from the nucleic acid of interest.

The recombinant expression vectors may also contain genes which encode a fusion moiety which provides increased expression of the recombinant protein; increased solubility of the recombinant protein; and aid in the purification of a target recombinant protein by acting as a ligand in affinity purification. For example, a proteolytic cleavage site may be added to the target recombinant protein to allow separation of the recombinant protein from the fusion moiety subsequent to purification of the fusion protein.

Recombinant expression vectors can be introduced into host cells to produce a transformed host cell. Accordingly, the disclosure includes a host cell comprising a recombinant expression vector. The term "transformed host cell" is intended to include prokaryotic and eukaryotic cells which have been transformed or transfected with a recombinant expression vector. The terms "transformed with", "transfected with", "transformation" and "transfection" are intended to encompass introduction of nucleic acid (e.g. a vector) into a cell by one of many possible techniques known in the art. Prokaryotic cells can be transformed with nucleic acid by, for example, electroporation or calcium-chloride mediated transformation. Nucleic acid can be introduced into mammalian cells via conventional techniques such as calcium phosphate or calcium chloride co-precipitation, DEAE-dextran-mediated transfection, lipofectin, electroporation or microinjection. Suitable methods for transforming and transfecting host cells can be found in Sambrook et al. (Molecular Cloning: A Laboratory Manual, 2nd Edition, Cold Spring Harbor Laboratory press (1989)), and other such laboratory textbooks.

Suitable host cells include a wide variety of prokaryotic and eukaryotic host cells. For example, the SARS-related proteins may be expressed in bacterial cells such as E. coli, Pseudomonas, Bacillus subtillus, insect cells (using baculovirus), yeast cells or mammalian cells. Other suitable host cells can be found in Goeddel, Gene Expression Technology: Methods in Enzymology 185, Academic Press, San Diego, CA (1991).

SARS-related proteins may also be isolated from a coronavirus or from a sample from a patient with SARS. The protein may be purified by conventional purification methods known to those in the art, such as chromatography methods, high performance liquid chromatography methods or precipitation.

For example, an anti-SARS-related protein antibody (as described below) may be used to isolate a SARS-related protein, which is then purified by standard methods.

The proteins may also be prepared by chemical synthesis using techniques well known in the chemistry of proteins such as solid phase synthesis (Merrifield, 1964, J. Am. Chem. Assoc. 85:2149-2154) or synthesis in homogenous solution (Houbenweyl, 1987, Methods of Organic Chemistry, ed. E. Wansch, Vol. 15 I and II, Thieme, Stuttgart).

### II. Uses

The present disclosure includes all uses of the SARS-related proteins including, but not limited to, the preparation of antibodies, the isolation of substances that modulate SARS-related protein expression and/or activity as well as the use of the proteins and modulators thereof in diagnostic, biomedical, therapeutic and vaccine applications. Some of the uses are further described below.

### (a) Antibodies

The isolation of a SARS-related protein enables the preparation of antibodies specific for the virus, likely coronavirus, that is causative of SARS. Accordingly, the present disclosure provides an antibody that binds to a SARS-related protein. Antibodies can be prepared which bind a distinct epitope in an unconserved region of the SARS-related protein. An unconserved region of the SARS-related protein is one that does not have substantial sequence homology to other proteins, for example, other related viral proteins which have at least one homologous region to a region of said SARS-related protein.

Antibodies to the SARS-related protein may also be prepared using techniques known in the art. For example, by using a peptide of the SARS-related protein, polyclonal antisera or monoclonal antibodies can be made using standard methods. A mammal, (e.g., a mouse, hamster, or rabbit) can be immunized with an immunogenic form of the peptide which elicits an antibody response in the mammal. Techniques for conferring immunogenicity on a peptide include conjugation to carriers or other techniques well known in the art. For example, the protein or peptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be obtained and, if desired, polyclonal antibodies isolated from the sera.

To produce monoclonal antibodies, antibody producing cells (lymphocytes) can be harvested from an immunized animal and fused with myeloma cells by standard somatic cell fusion procedures thus immortalizing these cells and yielding hybridoma cells. Such techniques are well known in the art, (e.g., the hybridoma technique originally developed by Kohler and Milstein (Nature 256, 495-497 (1975)) as well as other techniques such as the human B-cell hybridoma technique (Kozbor et al., Immunol. Today 4, 72 (1983)), the EBV-hybridoma technique to produce human monoclonal antibodies (Cole et al. Monoclonal Antibodies in Cancer Therapy (1985) Allen R. Bliss, Inc., pages 77-96), and screening of combinatorial antibody libraries (Huse et al., Science 246, 1275 (1989)). Hybridoma cells can be screened immunochemically for production of antibodies specifically reactive with the peptide and the monoclonal antibodies can be isolated. Therefore, the disclosure also contemplates hybridoma cells secreting monoclonal antibodies with specificity for a SARS-related protein as described herein.

The term "antibody" as used herein is intended to include fragments thereof which also specifically react with a SARS-related protein or fragments thereof. Antibodies can be fragmented using conventional techniques and the fragments screened for utility in the same manner as described above. For example, F(ab')2 fragments can be generated by treating antibody with pepsin. The resulting F(ab')2 fragment can be treated to reduce disulfide bridges to produce Fab' fragments.

Chimeric antibody derivatives, i.e., antibody molecules that combine a non-human animal variable region and a human constant region are also contemplated within the scope of the disclosure. Chimeric antibody molecules can include, for example, the antigen binding domain from an antibody of a mouse, rat, or other species, with human constant regions. Conventional methods may be used to make chimeric antibodies containing the immunoglobulin variable region which recognizes the gene product of SARS-related protein antigens (See, for example, Morrison et al., Proc. Natl Acad. Sci. U.S.A. 81,6851 (1985); Takeda et al., Nature 314, 452 (1985), Cabilly et al., U.S. Patent No. 4,816,567; Boss et al., U.S. Patent No. 4,816,397; Tanaguchi et al., European Patent Publication EP171496; European Patent Publication 0173494, United Kingdom patent GB 2177096B). It is expected that chimeric antibodies would be less immunogenic in a human subject than the corresponding non-chimeric antibody.

Monoclonal or chimeric antibodies specifically reactive with a protein as described herein can be further humanized by producing human constant region chimeras, in which parts of the variable regions, particularly the conserved framework regions of the antigen-binding domain, are of human origin and only the hypervariable regions are of non-human origin. Such immunoglobulin molecules may be made by techniques known in the art, (e.g., Teng et al., Proc. Natl. Acad. Sci. U.S.A., 80, 7308-7312 (1983); Kozbor et al., Immunology Today, 4, 7279 (1983); Olsson et al., Meth. Enzymol., 92, 3-16 (1982)), and PCT Publication WO92/06193 or EP 0239400). Humanized antibodies can also be commercially produced (Scotgen Limited, 2 Holly Road, Twickenham, Middlesex, Great Britain.)

Specific antibodies, or antibody fragments, such as, but not limited to, single-chain Fv monoclonal antibodies reactive against SARS-related proteins may also be generated by screening expression libraries encoding immunoglobulin genes, or portions thereof, expressed in bacteria with peptides produced from the nucleic acid molecules of SARS-related proteins. For example, complete Fab fragments, VH regions and FV regions can be expressed in bacteria using phage expression libraries (See for example Ward et al., Nature 341, 544-546: (1989); Huse et al., Science 246, 1275-1281 (1989); and McCafferty et al. Nature 348, 552-554 (1990)). Alternatively, a SCID-hu mouse, for example the model developed by Genpharm, can be used to produce antibodies or fragments thereof.

### (b) Diagnostic Assays

The finding by the present inventors of novel SARS-related proteins allows the development of diagnostic assays for SARS.

In one embodiment , the SARS-related protein, or functional fragment thereof, can be used in a method to detect antibodies in a sample from patients suspected of having SARS. Accordingly, the present disclosure provides a method for detecting SARS in a sample comprising contacting the sample with a SARS-related protein which is capable of being detected after it becomes bound to an antibody to SARS-related protein in the sample. These methods include, for example, enzyme linked immunosorbent assay (ELISA), radioimmunoassays (RIA), immunoradiometric assays (IRMA) and immunoenzymatic assays (IEMA). All of these detection methods are well known in the art (for example see US Patent 4,376,110 or US Patent 4,486,530). In this context a "functional fragment" includes a fragment of the SARS-related protein that can bind to antibodies found in a sample taken from a patient infected with coronavirus.

In a specific embodiment, the diagnostic assay will use the nucleocapsid protein, or a fragment thereof, as shown in Figure 1 as the nucleocapsid protein appears to be a major immunogenic antigen of the coronavirus.

In another embodiment, a sample from a patient may be assayed for the presence of the SARS-related protein. The SARS-related protein may be detected in a sample using antibodies that bind to the SARS-related protein which are described in detail in Section II(a). Accordingly, the present disclosure provides a method for detecting SARS in a sample comprising contacting the sample with an antibody that binds to a SARS-related protein which is capable of being detected after it becomes bound to the SARS-related protein in the sample.

Antibodies specifically reactive with SARS-related proteins, or derivatives thereof, such as enzyme conjugates or labeled derivatives, may be used to detect SARS-related proteins in various biological materials, for example they may be used in any known immunoassays which rely on the binding interaction between an antigenic determinant of SARS-related protein, and the antibodies. Examples of such assays are radioimmunoassays, enzyme immunoassays (e.g. ELISA), immunofluorescence, immunoprecipitation, latex agglutination, hemagglutination and histochemical tests. Thus, the antibodies may be used to detect and quantify SARS-related proteins in a sample in order to determine its role in particular cellular events or pathological states, and to diagnose and treat such pathological states.

Cytochemical techniques known in the art for localizing antigens using light and electron microscopy may be used to detect SARS-related proteins. Generally, an antibody of the invention may be labelled with a detectable substance and SARS-related protein may be localised in tissue based upon the presence of the detectable substance. Examples of detectable substances include various enzymes, fluorescent materials, luminescent materials and radioactive materials. Examples of suitable enzymes include horseradish peroxidase, biotin, alkaline phosphatase, β-galactosidase, or acetylcholinesterase; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of suitable radioactive material include radioactive iodine I-125, I-131 or 3-H. Antibodies may also be coupled to electron dense substances, such as ferritin or colloidal gold, which are readily visualised by electron microscopy.

Indirect methods may also be employed in which the primary antigen-antibody reaction is amplified by the introduction of a second antibody, having specificity for the antibody reactive against the SARS-related protein. By way of example, if the antibody having specificity against the SARS-related protein is a rabbit IgG antibody, the second antibody may be goat anti-rabbit gamma-globulin labelled with a detectable substance as described herein.

Where a radioactive label is used as a detectable substance, the SARS-related protein may be localized by autoradiography. The results of autoradiography may be quantitated by determining the density of particles in the autoradiographs by various optical methods, or by counting the grains.

### (c) Modulators of the SARS-related protein

In addition to antibodies described above, other substances that modulate SARS-related protein expression or activity can be identified. Accordingly, the present disclosure includes the use of the SARS-related proteins to develop or identify substances that modulate SARS-related protein expression or activity. Such substances may be useful in the therapeutic applications to treat SARS which are described below.

### (i) Substances that bind SARS-related proteins

Substances that affect SARS-related proteins activity can be identified based on their ability to bind to a SARS-related proteins.

Substances which can bind with the SARS-related proteins may be identified by reacting the SARS-related proteins with a substance which potentially binds to SARS-related proteins, and assaying for complexes, for free substance, or for non-complexed SARS-related proteins, or for activation of SARS-related proteins. In particular, a yeast two hybrid assay system may be used to identify proteins which interact with SARS-related proteins (Fields, S. and Song, O., 1989, Nature, 340:245-247). Systems of analysis which also may be used include ELISA.

Accordingly, the disclosure provides a method of identifying substances which can bind with a SARS-related protein, comprising the steps of:
(a) reacting a SARS-related protein and a test substance, under conditions which allow for formation of a complex between the SARS-related proteins and the test substance, and
(b) assaying for complexes of a SARS-related protein and the test substance, for free substance or for non complexed a SARS-related protein, wherein the presence of complexes indicates that the test substance is capable of binding a SARS-related protein.

The SARS-related proteins used in the assay may have the amino acid sequence shown in Figure 1 or 2 or may be a fragment, analog, derivative, homolog or mimetic thereof as described herein.

Conditions which permit the formation of substance and SARS-related protein complexes may be selected having regard to factors such as the nature and amounts of the substance and the protein.

The substance-protein complex, free substance or non-complexed proteins may be isolated by conventional isolation techniques, for example, salting out, chromatography, electrophoresis, gel filtration, fractionation, absorption, polyacrylamide gel electrophoresis, agglutination, or combinations thereof. To facilitate the assay of the components, antibody against a SARS-related protein or the substance, or labelled SARS-related protein, or a labelled substance may be utilized. The antibodies, proteins, or substances may be labelled with a detectable substance as described above.

The SARS-related protein, or the substance used in the method may be insolubilized. For example, the SARS-related protein or substance may be bound to a suitable carrier. Examples of suitable carriers are agarose, cellulose, dextran, Sephadex, Sepharose, carboxymethyl cellulose polystyrene, filter paper, ion-exchange resin, plastic film, plastic tube, glass beads, polyamine-methyl vinyl-ether-maleic acid copolymer, amino acid copolymer, ethylene-maleic acid copolymer, nylon, silk, etc. The carrier may be in the shape of, for example, a tube, test plate, beads, disc, sphere etc.

The insolubilized protein or substance may be prepared by reacting the material with a suitable insoluble carrier using known chemical or physical methods, for example, cyanogen bromide coupling.

The proteins or substance may also be expressed on the surface of a cell using the methods described herein.

The disclosure also contemplates assaying for an antagonist or agonist of the action of a SARS-related protein.

### (ii) Peptide Mimetics

The present disclosure also includes peptide mimetics of the SARS-related proteins. For example, a peptide derived from a binding domain of a SARS-related protein will interact directly or indirectly with an associated molecule in such a way as to mimic the native binding domain. Such peptides may include competitive inhibitors, enhancers, peptide mimetics, and the like. All of these peptides as well as molecules substantially homologous, complementary or otherwise functionally or structurally equivalent to these peptides may be used for purposes of the present disclosure.

"Peptide mimetics" are structures which serve as substitutes for peptides in interactions between molecules (See Morgan et al (1989), Ann. Reports Med. Chem. 24:243-252 for a review). Peptide mimetics include synthetic structures which may or may not contain amino acids and/or peptide bonds but retain the structural and functional features of a peptide, or enhancer or inhibitor. Peptide mimetics also include peptoids, oligopeptoids (Simon et al (1972) Proc. Natl. Acad, Sci USA 89:9367); and peptide libraries containing peptides of a designed length representing all possible sequences of amino acids corresponding to a peptide.

Peptide mimetics may be designed based on information obtained by systematic replacement of L-amino acids by D-amino acids, replacement of side chains with groups having different electronic properties, and by systematic replacement of peptide bonds with amide bond replacements. Local conformational constraints can also be introduced to determine conformational requirements for activity of a candidate peptide mimetic. The mimetics may include isosteric amide bonds, or D-amino acids to stabilize or promote reverse turn conformations and to help stabilize the molecule. Cyclic amino acid analogues may be used to constrain amino acid residues to particular conformational states. The mimetics can also include mimics of inhibitor peptide secondary structures. These structures can model the 3-dimensional orientation of amino acid residues into the known secondary conformations of proteins. Peptoids may also be used which are oligomers of N-substituted amino acids and can be used as motifs for the generation of chemically diverse libraries of novel molecules.

Peptides may also be used to identify lead compounds for drug development. The structure of the peptides described herein can be readily determined by a number of methods such as NMR and X-ray crystallography. A comparison of the structures of peptides similar in sequence, but differing in the biological activities they elicit in target molecules can provide information about the structure-activity relationship of the target. Information obtained from the examination of structure-activity relationships can be used to design either modified peptides, or other small molecules or lead compounds that can be tested for predicted properties as related to the target molecule. The activity of the lead compounds can be evaluated using assays similar to those described herein.

Information about structure-activity relationships may also be obtained from co-crystallization studies. In these studies, a peptide with a desired activity is crystallized in association with a target molecule, and the X-ray structure of the complex is determined. The structure can then be compared to the structure of the target molecule in its native state, and information from such a comparison may be used to design compounds expected to possess.

### (iii) Drug Screening Methods

In accordance with one embodiment, the disclosure enables a method for screening candidate compounds for their ability to modulate (e.g. increase or decrease) the activity of a SARS-related protein. Such compounds may be useful in treating SARS. The method comprises providing an assay system for assaying SARS-related protein activity, assaying the activity in the presence or absence of the candidate or test compound and determining whether the compound has increased or decreased SARS-related protein activity.

Accordingly, the present disclosure provides a method for identifying a compound that affects SARS-related protein activity or expression comprising:
(a) incubating a test compound with a SARS-related protein or a nucleic acid encoding a SARS-related protein; and
(b) determining an amount of SARS-related protein activity or expression and comparing with a control (i.e. in the absence of the test substance), wherein a change in the SARS-related protein activity or expression as compared to the control indicates that the test compound has an effect on SARS-related protein activity or expression.

In accordance with a further embodiment, the disclosure enables a method for screening candidate compounds for their ability to increase or decrease expression of a SARS-related protein. The method comprises putting a cell with a candidate compound, wherein the cell includes a regulatory region of a SARS-related protein gene operably joined to a reporter gene coding region, and detecting a change in expression of the reporter gene.

In one embodiment, the present disclosure provides culture systems in which cell lines which express the SARS-related protein gene, and thus SARS-related protein products, are incubated with candidate compounds to test their effects on SARS-related protein expression. Such culture systems can be used to identify compounds which upregulate or downregulate SARS-related protein expression or its function, through the interaction with other proteins.

In another embodiment, the present disclosure provides microchips that have nucleic acid molecules encoding SARS-related protein attached thereto. Such microchips are useful in high throughput screening assays for identifying drugs that interact with SARS-related protein.

Such compounds can be selected from protein compounds, chemicals and various drugs that are added to the culture medium. After a period of incubation in the presence of a selected test compound(s), the expression of SARS-related protein can be examined by quantifying the levels of SARS-related protein mRNA using standard Northern blotting procedure, as described in the examples included herein, to determine any changes in expression as a result of the test compound. Cell lines transfected with constructs expressing SARS-related protein can also be used to test the function of compounds developed to modify the protein expression. In addition, transformed cell lines expressing a normal SARS-related protein could be mutagenized by the use of mutagenizing agents to produce an altered phenotype in which the role of mutated SARS-related protein can be studied in order to study structure/function relationships of the protein products and their physiological effects.

In the identification or design of potential drugs, the biologically active protein SARS-related protein, or fragment thereof, is used to identify small molecules with which they interact in order to fashion drugs which will interfere with the function of the polypeptide *in vivo.* In one approach, one first determines the three dimensional structure of the protein of interest or, for example, of the SARS-related protein receptor or ligand complex, by x-ray crystallography, by computer modelling, or most typically, by a combination of approaches. Less frequently, useful information regarding the structure of the polypeptide can be gained by modelling based on structure of homologous proteins. Thus, by one of these methods, one may design drugs which act as agonists or antagonists of SARS-related protein polypeptide activity. These inhibitors can be small molecules, antibodies, monoclonal antibodies, or antibody fragments. Said inhibitors can therefore then be used to treat SARS and coronavirus mediated diseases.

This disclosure also contemplates the use of competitive drug screening assays in which neutralizing antibodies capable of specifically binding the SARS-related protein compete with a test compound for binding to the SARS-related protein or fragment thereof. In this manner, the antibodies can be used to detect the presence of any polypeptide which shares one or more antigenic determinants of the SARS-related protein. Antibodies can be prepared according to well known methods (Hum and Chantler, 1980). A peptide, or a fragment thereof, is generally injected over a several month period into rabbits or other suitable animal. The sera is tested for immunoreactivity to the SARS-related protein, or fragment thereof.

Once a potential drug or SARS-related protein modulator is identified using the above screening assays, it can be tested for therapeutic efficacy in a variety of *in vitro* or *in vivo* models.

### (e) Therapeutic Uses

As previously discussed, the SARS-related proteins were isolated from a patient with SARS and likely are derived from a coronavirus or coronavirus-like virus. Accordingly, the present disclosure includes the use of SARS-related proteins and modulators thereof in therapeutic applications including the treatment or prevention of conditions associated with coronavirus. The definitions used in this section apply to all embodiments unless otherwise noted.

In one embodiment, the present disclosure provides a method of treating or preventing SARS comprising administering to a cell or animal in need thereof, an effective amount of agent that modulates SARS-related protein expression and/or activity. The disclosure also provides a use of an effective amount of an agent that modulates SARS-related protein expression and/or activity to treat SARS or in the manufacture of a medicament to treat SARS.

The term "agent that modulates SARS-related protein expression and/or activity" means any substance that can alter the expression and/or activity of SARS-related protein and includes agents that can inhibit SARS-related protein expression or activity (SARS-related protein antagonists) and agents that can enhance SARS-related protein expression or activity (SARS-related protein agonists). The agent can be any type of molecule including, but not limited to, proteins, peptides, nucleic acids (including DNA, RNA, antisense oligonucleotides, peptide nucleic acids), carbohydrates, organic compounds, small molecules, natural products and library extracts. Examples of agents which may be used to modulate SARS-related protein include nucleic acid molecules encoding SARS-related protein; the SARS-related protein as well as fragments, analogs, derivatives or homologs thereof; antibodies; antisense nucleic acids; peptide mimetics; substances isolated using the screening methods described herein.

The term "effective amount" as used herein means an amount effective, at dosages and for periods of time necessary to achieve the desired results.

The term "animal" as used herein includes all members of the animal kingdom, including humans.

The term "treatment or treating" as used herein means an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treating" can also mean prolonging survival as compared to expected survival if not receiving treatment.

In one embodiment, the disclosure provides a method of treating or preventing SARS comprising administering an effective amount of a SARS-related protein antagonist. The present disclosure also provides a use of an effective amount of a SARS-related protein antagonist to treat SARS or in the manufacture of a medicament to treat SARS.

The term "SARS-related protein antagonist" includes any substance that can inhibit the expression of a SARS-related protein gene and/or the activity of a SARS-related protein. Examples of SARS-related protein antagonists include antibodies as described above.

In a specific embodiment, the antagonist will bind or interfere with the activity of the spike glycoprotein shown in Figure 2.

### (f) Pharmaceutical Compositions

The above described substances including nucleic acids encoding SARS-related proteins, SARS-related proteins, antibodies, as well as other agents that modulate SARS-related proteins may be formulated into pharmaceutical compositions for administration to subjects in a biologically compatible form suitable for administration *in vivo*. By "biologically compatible form suitable for administration *in vivo"* is meant a form of the substance to be administered in which any toxic effects are outweighed by the therapeutic effects. The substances may be administered to living organisms including humans, and animals.

Administration of a therapeutically active amount of pharmaceutical compositions of the present disclosure is defined as an amount effective, at dosages and for periods of time necessary to achieve the desired result. For example, a therapeutically active amount of a substance may vary according to factors such as the disease state, age, sex, and weight of the individual, and the ability of the substance to elicit a desired response in the individual. Dosage regimes may be adjusted to provide the optimum therapeutic response. For example, several divided doses may be administered daily or the dose may be proportionally reduced as indicated by the exigencies of the therapeutic situation.

An active substance may be administered in a convenient manner such as by injection (subcutaneous, intravenous, etc.), oral administration, inhalation, transdermal application, or rectal administration. Depending on the route of administration, the active substance may be coated in a material to protect the compound from the action of enzymes, acids and other natural conditions which may inactivate the compound. If the active substance is a nucleic acid encoding a SARS-related protein it may be delivered using techniques known in the art.

The compositions described herein can be prepared by *per se* known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985) or Handbook of Pharmaceutical Additives (compiled by Michael and Irene Ash, Gower Publishing Limited, Aldershot, England (1995)). On this basis, the compositions include, albeit not exclusively, solutions of the substances in association with one or more pharmaceutically acceptable vehicles or diluents, and may be contained in buffered solutions with a suitable pH and/or be iso-osmotic with physiological fluids. In this regard, reference can be made to U.S. Patent No. 5,843,456. As will also be appreciated by those skilled, administration of substances described herein may be by an inactive viral carrier.

### (g) Vaccines

The present disclosure includes vaccines for treating or preventing coronavirus mediated diseases.

In one embodiment, the vaccine comprises a SARS-related protein in admixture with a suitable diluent or carrier. In another embodiment the vaccine comprises a nucleic acid encoding a SARS-related protein in admixture with a suitable diluent or carrier. The term "SARS-related protein" is as previously defined herein to include all homologs, analogs, fragments, or derivatives of the SARS-related protein from coronavirus including the proteins shown in Figures 1 and 2. The fragments of the SARS-related protein will be sufficient in order to elicit an immune response against the coronavirus.

In a specific embodiment, the vaccine will comprise the spike glycoprotein of Figure 2 or a fragment thereof. The spike glycoprotein plays an important role in the initial stages of the infection by mediating the attachment of the virus to the cell membrane of the host. Consequently, inhibiting or neutralizing this protein may be an effective therapeutic strategy.

In a further embodiment, the vaccine comprises an antibody that binds to a SARS-related protein in admixture with a suitable diluent or carrier. Such antibodies can be prepared as hereinbefore described.

The vaccine can either include a protein (such as a SARS-related protein or antibody that binds a SARS-related protein or peptide) or a nucleic acid encoding the SARS-related protein. Such nucleic acids include free or naked RNA or DNA or in a vector. In a preferred embodiment, the nucleic acid sequence is contained in a vector or plasmid. In one embodiment, the vector may be viral such as poxvirus, adenovirus or alphavirus. Preferably the viral vector is incapable of integration in recipient animal cells. The elements for expression from said vector may include a promoter suitable for expression in recipient animal cells.

The vaccines may be in admixture with a suitable carrier, diluent, or excipient such as sterile water, physiological saline, glucose or the like to form suitable vaccine formulations. The vaccines can also be lyophilized. The vaccines may also contain auxiliary substances such as wetting or emulsifying agents, pH buffering agents, gelling or viscosity enhancing additives, preservatives, flavoring agents, colors, and the like, depending upon the route of administration and the preparation desired. In this regard, reference can be made to U.S. Patent No. 5,843,456. Reference can also be made to the textbook Vaccine Design: the Subunit and Adjuvant Approach, Michael F.Powell and Mark J. Newman, eds. Plenum Press, New York, 1995.

The vaccine may additionally include adjuvants to enhance the immune response to the SARS-related protein or antibody. A wide range of extrinsic adjuvants can provoke potent immune responses to antigens. These include saponins complexed to membrane protein antigens (immune stimulating complexes), pluronic polymers with mineral oil, killed mycobacteria and mineral oil, Freund's complete adjuvant, bacterial products such as muramyl dipeptide (MDP) and lipopolysaccharide (LPS), as well as lipid A, and liposomes.

Accordingly, the present disclosure provides a method of eliciting an immune response to coronavirus comprising administering an effective amount of a vaccine formulation comprising a SARS-related protein, a nucleic acid encoding a SARS-related protein or an antibody to a SARS-related protein to an animal in need thereof.

The term "eliciting an immune response" is defined as causing, enhancing, or improving any response of the immune system, for example, of either a humoral or cell-mediated nature. Whether a vaccine or antigen elicits an immune response can be assessed using assays known to those skilled in the art including, but not limited to, antibody assays (for example ELISA assays), antigen specific cytotoxicity assays and the production of cytokines (for example ELISPOT assays).

The term "administering" is defined as any conventional route for administering an antigen to an animal for use in the vaccine field as is known to one skilled in the art. This may include, for example, administration via the topical, oral and parenteral (i.e. subcutaneous, intradermal, intramuscular, etc.) routes and further includes, transdermal and mucosal delivery, including mucosal delivery accomplished by oral feeding, inhaling and through the membranes accessible through the terminal portions of the large intestine.

Also provided are kits for carrying out the SARS detection assays discussed above. For example, such kits may include any one or more of the following: an isolated SARS-related polypeptide, one or more antibodies specific to a SARS-related polypeptide, a detection label and instructions for using the kit. As will be appreciated by one of skill in the art, other suitable components and reagents may also be included. It is of note that the above-described kit may be used for detecting the presence of anti-SARS antibodies in a sample (using labeled isolated SARS-related polypeptide or immunogenic fragments thereof) or for detecting the presence of SARS-related polypeptides.

The following non-limiting examples are illustrative of the present invention:

### EXAMPLE 1

### EXPERIMENTAL PROCEDURES

### Discovery and Isolation of SARS coronavirus

SARs coronavirus was grown in Vero E6 cell culture using Minimal Essential Medium (designated MEM) supplemented with antibiotics, fungizone, amino acids, vitamins, HEPES buffer and 3% fetal calf serum. Medium was stored at 4°C.

Confluent monolayers of Vero-E6 cells grown in culture tubes were inoculated with patient respiratory secretion specimens were incubated at 37°C and examined under an inverted microscope daily. Observation of a cytopathic effect in some of the inoculated cultures (as compared to normal healthy cella) indicated the possibility of virus growth.

All bacteriology was negative. Identification of the virus as a new cornonavirus was done by PCR tests for most known families of viruses. This excluded many agents as the cause of disease, especially viruses or bacteria known to be asscoiated with pneumonia. However, there was a positive reaction for coronavirus 5 out of 8 of the initial cluster of SARS. Sequencing of the PCR products gave a sequence confirming that a coronavirus was the probable aetiological agent, and that the SARS coronavirus is a new family of virus that is distantly related to previously known.human and animal coronaviruses. Employing the Vero-E6 grown SARS coronavirus antigen, serological tests (ELISA and IFA) to detect the presence of antibodies in patients were developed. Employing these methods, further testing confirmed the SARS coronavirus as the cause of the disease. In more than 250 suspect or probable SARS cases, 95% tested positive for this new coronavirus.

### Nucleocapsid isolations, RNA purification and extraction from SARS coronavirus:

Vero E6 cells were inoculated with SARS coronavirus at a mutiplicity of between 1 and 5 infectious units per cell. When approximately 80% of the SARS CoV infected cells showed CPE (3 days post-infection), they were harvested for viral nucleocapsid isolation. The SARS CoV nucleocapsid were isolated from infected cells as follows. Briefly, 10⁷ infected cells were lysed in 6mL lysis buffer (0.5% deoxycholate, 0.5% NP-40, 30mM Tris pH 7.5, 5mM Mg(C2H3O2)2.4H2O, 125mM KCl, 0.5mM EDTA, 3.6mM CaCl2, 6mM 2-mercaptoethanol) and sonicated (Branson Sonifier 250) on ice at 50% power for 10, 10 second bursts. Cell lysis was confirmed by light microscopy on a hemocytometer. 600 units of DNase I (Invitrogen, Burlington, Ontario) and 6µl of 10mg/mL RNase A (Sigma, Oakville, Ontario) were added and the mixture was incubated at 30C for 90 minutes with 600 units of DNase I added every 30 minutes. 6mL of 1,1,2-trichloro-trifluoroethane (Sigma, Oakville, Ontario) was added and mixed vigorously before centrifugation at 800 xg for 10 minutes. The top fraction was removed and ultracentrifuged (Beckman Optima XL-100K) through a 5 to 40% glycerol gradient in lysis buffer at 151,000 xg for 1 hour at 4C. The RNA was then extracted from the nucleocapsid preparation using Qiagen's RNeasy Kit as follows:
1. Resuspend pellet in 100 ul PBS and add to 350 ul RTL (with betamercaptoethanol.) for RNA extraction and polyA RNA) for RNA extraction.
2. Add 450 ul 70% EtOH and vortex.
3. Attach purification column to vacuum manifold and set vacuum to 600 psi)
4. Add sample to column and apply vacuum until the entire sample has passed through
5. add 700 ul RW1 buffer and apply vacuum.
6. Add 500 ul RPE buffer and apply vacuum
7. Add 500 ul RPE buffer and apply vacuum (i.e. 2 washes)
8. Transfer column to a collection tube and centrifuge for 2 min. at 10 K rpm to remove traces of RPE.
9. Transfer column to 1.5 ml eppendorf tube, and add 50 ul DEPC water to the column membrane. Let stand 1 min.
10. Centrifuge for 1 min. at 10 K rpm and store RNA at -70C for later use in sequencing. The purity and yield of the viral RNA was confirmed by checked by spectrophotometry

### VERO E6 Cell Growth Media

### Reagents:

Fetal Bovine Serum Gibco Cat #16000-044
Minimum Essential Medium (MEM) with Earle's salts, without L-glutamine Gibco BRL Cat# 11090-081
Penicillin-Streptomycin-Glutamine 100x, liquid (10,000 U/ml penicillin G; 10,000 ug/ml
streptomycin sulfate and 29.2 mg. of L-Glutamine) Gibco BRL Cat# 10378-057
HEPES Buffer, 1 M stock solution Gibco BRL Cat# 15630-080
MEM Sodium Pyruvate Solution 100 mM (100x), liquid Gibco BRL Cat# 11360-070
MEM Non-Essential Amino Acids Solution 10mM (100 x), liquid Gibco BRL Cat# 11140-050

### To prepare 500 ml of cell growth media:

| Reagent | Volume | Final Concentration |
|---|---|---|
| Fetal Calf Serum | 50 ml | 10% |
| Penicillin-Streptomycin- | 5.0 ml | 100 U/ml |
| Penicillin and Glutamine | | 100 ug/ml |
| Streptomycin | | |
| | | .292 mg/ml |
| Glutamine | | |
| HEPES buffer | 12.5 ml | 25 mM |
| MEM Non essential AA | 5 ml | 0.1 mM |
| MEM Sodium pyruvate | 5 ml | 1.0 mM |

*Preparation of the Primary Material at NML -* As discussed above, clinical specimens obtained from the original case cluster were extensively investigated for the presence of bacterial and viral pathogens (1). Nasopharyngeal swab and bronchoalveolar lavage fluids from several of these patients were found to be positive by reverse transcription-polymerase chain reaction (RT-PCR) for human metapneumovirus and the novel coronavirus (1). Inoculation of the bronchoalveolar lavage fluid from the 43-year-old male patient in Vero E6 cells produced a strong cytopathic effect on day four after infection. The second passage of this viral isolate was further used to produce large quantities of the virus. Initially this virus material was used to assess its antigenicity with convalescent serum samples from SARS patients. The convalescent sera that were previously found to be positive for antibodies to the virus by indirect immunofluorescence assay (IFA) (11) strongly reacted in Western blot with a ∼46 kDa protein (Fig. 3A) similar in size to the nucleocapsid protein of coronaviruses (12).

In order to prepare this, (and perhaps other SARS-related proteins) for proteolytic digestion, the virus was purified by 20-60% linear sucrose gradient. Western blotting of the gradient fractions showed that fraction #4 (density 1.18g/cm 3 ) reacted strongly with a convalescent serum from a SARS patient. This fraction was run on SDS-PAGE and stained with Coomassie blue (Fig. 3B), then excised and transferred to the University laboratories for digestion with various proteolytic enzymes.
*Proteolytic Digestions -* The excised protein bands were in-gel digested with one of three different enzymes [sequencing grade modified trypsin (Promega), Lys-C, or Asp-N (both from Roche Biochemical)]. Digestions were performed according to the procedure described by Shevchenko et al (13) either in H₂ ¹⁶O, or in a 1:1 H₂ ¹⁶O:H₂ ¹⁸O mixture (14-16), prepared from 98% H₂ ¹⁸O (Isotec). Unless otherwise noted, all other chemicals were purchased from Sigma.

The extracts containing the peptide mixture were lyophilized and resuspended in 5.5 ml of 0.5 % TFA in water. 0.5 ml of the resulting sample was mixed 1:1 with 2,5-dihydroxybenzoic acid (DHB, 150 mg/ml in water:acetonitrile 1:1) matrix solution, and deposited on the gold surface of a MALDI target. The remaining 5 ml was used for mHPLC-MALDI -QqTOF analysis.

*Chromatography -* Chromatographic separations were performed using an Agilent 1100 Series system. Deionized (18 MΩ) water and HPLC-grade acetonitrile were used for the preparation of eluents. Samples (5 µl) were injected onto a 150mm ¥ 150 mm column (Vydac 218 TP C18, 5m) and eluted with a linear gradient of 1-80% acetonitrile (0.1% TFA) in 60 min. The column effluent (4 ml/min) was mixed on-line with DHB matrix solution (0.5 µl/min) and deposited by a small computer-controlled robot onto a movable gold target at 1 min intervals (17). The vast majority of the tryptic fragments were eluted within 40 min under the HPLC condition used, so 40 fractions were normally collected.

*Glycoprotein Analysis -* The original intention was to postpone any detailed analysis of the higher mass protein to a subsequent investigation. When the inventors decided to include this effort in the present measurements, the only materials that they had available were two lyophilized samples from digests of the larger protein (∼180 kDa band), one from a tryptic digest and one from a Lys-C digest. The sample from the tryptic digest was separated by HPLC and used for analysis of the glycosylated peptides. The sample from the Lys-C digest was digested twice more, first by pNGase F (Roche Biochemical) to remove the sugars, then by trypsin to produce smaller fragments, (both digestions in H₂ ¹⁶O).

*Time-of-flight Mass Spectrometry -* The spots on the gold targets were analyzed individually, both by single mass spectrometry (MS) and by tandem mass spectrometry (MS/MS) in the Manitoba/Sciex prototype quadrupole/time-of-flight mass spectrometer (QqTOF) (18). Ions were produced by irradiation of the target with photon pulses from a 20-Hz nitrogen laser (Laser Science) with 300 mJ energy per pulse. Orthogonal injection of ions from the quadrupole into the TOF section normally produced a mass resolving power 10,000 FWHM and accuracy within a few mDa in the TOF instrument for both MS and MS/MS spectra, as long as the ion peak was reasonably intense.

### RESULTS

*Mass spectra from proteolytic digests of the ∼47-kDa Protein -* Figure 4A shows the *m*/*z* spectrum of the mixture of peptides resulting from tryptic digestion of the ∼47-kDa protein in H₂ ¹⁶O, before HPLC fractionation. A small region of this spectrum is shown in Fig. 4B, and an HPLC fraction showing some of the same ions is shown in Fig. 4C. Here the most intense ion in Fig. 5B has moved to a different fraction, but some of the weaker ions are much more prominent, so it is clear that individual peptide peaks are considerably easier to distinguish after HPLC separation. Spectra of the fractions are dramatically simpler and have a signal/noise ratio improved by a factor ∼10 or more.

Initial efforts to identify the protein, (based on database searching against the peptide fingerprint), failed to yield any significant matches, suggesting that it was a novel protein. *De novo* peptide sequencing was therefore undertaken in order to characterize it. For this purpose, samples were digested in the presence of a 50/50 mixture of H₂ ¹⁶O and H₂ ¹⁸O, as described above, since the addition of ¹⁸O or ¹⁶O during enzymatic cleavage identifies fragments containing the C-terminus by their distinctive isotopic patterns (14-16). In order to determine the amino acid sequence of the proteolytic fragments, each clearly observed peptide ion was selected in turn as a parent by the mass-selecting quadrupole of the QqTOF instrument, and subjected to collisionally-induced dissociation in the collision cell. For example, the resulting daughter ion spectrum from the *m*/*z* = 2297 parent is shown in Fig. 5, where the advantages of the ¹⁶O/¹⁸O addition for distinguishing the C- and N-terminal ions are clearly evident. The y ions, which contain the C-terminus, all show the doublet structure superimposed on the usual isotopic pattern, whereas the b ions, containing the N-terminus, have a normal pattern. A comparison between the measured m/z values and the masses calculated from the deduced sequence is given in Table 1.

Further examples are provided in the supplemental material. Fig. 8 shows the daughter ions from dissociation of the 1144 Da N-terminal peptide, indicating deletion of the N-terminal methionine and acetylation of the resulting N-terminal serine. Fig. 9 shows a comparison between the daughters of HPLC-separated parents of masses 1411 and 1795 Da, from tryptic and Lys-C digestions respectively. Fig. 10 shows a spectrum of the parent ion containing the C-terminus- the one C-terminal peptide that shows no doublet structure.

A comparison of experimental *m*/*z* values and masses calculated for the deduced sequences of all the peptides observed in tryptic digests is given in Table 2. In both tables 1 and 2, most observed m/z values and the masses calculated for the deduced amino acid sequences agree within ∼10 mDa, lending credibility to the assignments; the anomalously high values observed for a few ions in Table 1 correspond to peaks of very low intensity.

The MS and MS/MS measurements just described were applied first to the peptides resulting from tryptic digests of the gel band, listed in Table 1, and then to the products of a Lys-C digest. BLAST searching (20, 21) of the total Genbank protein database with these peptides was then undertaken in order to search for homology. The most definitive example was provided by the 2297 Da tryptic peptide. In that case, the highest rated results of the BLAST search are shown in Fig. 6; all are coronavirus nucleocapsid proteins, and all yield BLAST scores of 40 to 41, with E values 0.003. Moreover, the highest rated hit in the BLAST search that is not a coronavirus protein had a score of only 29 and a high E value of 9.4. Thus the ∼47 kDa protein is clearly a coronavirus nucleocapsid protein; there is complete agreement between the first 10 residues and those found by BLAST in a region of the coronaviruses that is highly conserved. On the other hand, only 3 out of the next 9 residues agree with any of the other viruses, so the SARS virus is significantly different from any of the other coronaviruses.. BLAST searches with the other peptides strengthened the evidence for significant differences between the SARS coronavirus and any other coronavirus in the database, but led to similar conclusions.

By April 12 these measurements had been carried out and most of them analyzed, yielding almost complete sequence information on the individual peptides, as summarized in Table 2. The task of fitting together the peptides was not yet done, however, since there were still a number of ambiguities in their order. To sort out this problem, an Asp-N digestion had also been carried out, (but not yet separated on the HPLC), and Glu-C and perhaps Arg-C digestions were planned as soon as sufficient material was available. More sophisticated BLAST searches (20) were also planned. However, these measurements and analyses turned out to be unnecessary, because at that stage a nucleotide sequence of infectious material, (also prepared by NML), was obtained by a group at the Michael Smith Genome Centre in Vancouver (Genbank database accession AY274119), soon followed by similar results from several other laboratories.

It soon became clear to us that the open reading frame identified by the Vancouver group as specifying the coronavirus nucleocapsid protein did in fact predict the aa sequence of the ∼47 kDa protein that we were analyzing, so we were able to remove the remaining ambiguities in ordering the proteolytic fragments listed in Table 2. A comparison of our results with the predicted sequence is shown in Fig. 11A; our mass spectral data cover more than 96% of the predicted sequence, and include both C-and N-termini. The mass spectra also indicate removal of the N-terminal methionine, and oxidation of all other methionines, as well as acetylation of the resulting N-terminal serine, as shown in Fig. 8. The N-terminal deletion and acetylation presumably occur as a result of post-translational modifications, which of course could not be predicted by the nucleotide data. Otherwise, our results are in complete agreement with the predicted sequence (Genbank database accession AY274119), a result consistent with the samples being derived from the same infectious source at NML.

*Mass spectra from proteolytic digests of the Spike Protein -* In addition to the almost completely defined ∼47 kDa protein, we have partially characterized a protein that appeared as a very weak band at an apparent mass ∼180 kDa in the gel separation (Fig. 3B). In spite of the low intensity, 39 peptides in the initial tryptic digest were found to belong to the ∼139 kDa "spike protein" predicted by the nucleotide sequence (Genbank database accession AY274119), and 36 of these were sufficiently intense for MS/MS measurements, which confirmed the identification (30% coverage). A summary of the data and the coverage for this protein is given in Table 5.

This protein is homologous to spike proteins in other coronaviruses, which are reputed to be extensively glycosylated, and to act as attachment proteins. Indeed, the predicted sequence of the spike protein of the SARS coronavirus contains 23 potential N-glycosylation sites (NXT or NXS), of which 17 are identified as likely sites by the Netglyc 1.0 server (21). (O-glycolsylation may also be possible, but has not been investigated here).

A tryptic digest of the spike protein was therefore treated with PNGase F to remove the glycans, as described above. This step converts asparagine residues to aspartic acids, thus specifying the corresponding deglycosylated peptides through observation of their mass differences of 0.984 Da per deglycosylated site from the values calculated from the predicted aa sequence. This procedure identified 9 glycopeptides and their deglycosylated products (Table 3), and raised the sequence coverage to 42%. MS/MS measurements on all deglycosylated peptides confirmed the predicted single N-glycosylation sites, and T111-126, T316-333 and T1140-1163 were found to have two glycosylation sites each (Table 3). For example, figure S5 shows MS and MS/MS spectra of the deglycosylated peptide T222-232. No peptides were observed containing possible sites that were not glycosylated, suggesting that some of the other sites may also be modified by glycosylation.

The tryptic digest of the spike protein without PNGase F deglycosylation yielded spectra of four of these glycopeptides that were sufficiently intense for detailed analysis, as summarized in Table 4. Microheterogeneity was observed in all four glycopeptides, and each glycoform was assigned an N-linked glycan composition. Two peptides, T222-232 and T778-796, were found to have high-mannose substitution MₓN_{y}, whereas the other two (T111-126, T226-287) showed complex glycan structures MₓN_{y}G_{z}F; (M=mannose, N=N-acetyl glucosamine, G=galactose, F=fucose), similar to the pattern observed in the bovine coronavirus hemagglutinin protein (22). These compositions each encompass more than one isomeric structure; some examples are given in Figure 7D. It is important to note that some observed glycoforms may result from in-source fragmentation, although the results suggest that this is improbable (see below).

Figure 7A shows the MALDI mass spectrum of the HPLC fraction containing glycosylated T111-126. This spectrum is interpreted in Table 3 as containing either one or two O-glycosylation sites; whereas peaks between *m*/*z* 3000 and 3800 show one possible glycosylation site, those higher than *m*/*z* 3800 possibly correspond to di-glycosylated T111-126 (see Fig. 5D). In Fig. 5B, MS of the glycosylated T222-232 HPLC fraction clearly highlights the presence of high mannose structures. Here the predominance of M₂N₉ , the highest possible form of N-linked high mannose oligosaccharide, suggests that there is little in-source fragmentation. Figure 7C is the tandem mass spectrum of T222-232 with an MgN₂ attachment. This spectrum shows losses of one to five mannose residues, loss of the whole oligosaccharide moiety (*m*/*z* 1257.733), loss of the whole moiety minus one N (*m*/*z* 1460.808), and loss of the carbohydrate residue via a cross-ring cleavage (m/z 1340.768). MS/MS analysis of m/z 3404.572, 3607.682 and 4093.818 ions confirmed the presence of complex glycan structures from observed losses of [G-N] moieties (not shown). Tandem MS was also performed on glycoforms of T226-287 and T778-796, with results consistent with the suggested complex glycan compositions in Table 4 (spectra not shown). All MS/MS spectra recorded in this study showed that the preferred fragmentation mode was loss of the entire sugar rather than loss of one residue at a time, which again argues against extensive in-source fragmentation.

Other candidate peptides were sought in their possible glycosylated forms but were not detected, perhaps because of the low amount of sample. Alternatively, they may not be detectable as *positive ions* because of the presence of negatively charged sialic acids. Indeed, the several galactosylated complex oligosaccharide compositions found in this study suggest the undetected presence of sialic acid, since the latter compound attaches to terminal galactose in such structures.

### DISCUSSION

*The nucleocapsid protein N -* Comparison of deduced amino acid sequences of different coronavirus N proteins revealed 32% identity between the SARS-related coronavirus and known viruses from the three coronavirus clusters. The phylogenetic tree (23) of the N protein-deduced amino acid sequences indicated that the SARS-related virus is only distantly related to any of the other clusters (Fig. 11 B). The evolutionarily distance, based on this phylogenetic tree analysis, makes it difficult to speculate about the origin of the virus.

Despite the striking heterogeneity of the SARS corona N protein when compared with other corona nucleoproteins certain domains seem functionally conserved (24). The SR-rich region of SARS N protein resembles that of murine and bovine corona viruses; in a short stretch of 36 residues (amino acids 176-212) it contains 14 serines and 7 arginines. The amino acid sequence in this region is highly variable among coronaviruses except for a core motif SRXX for which double or triple repeats are a distinguishable feature among all coronavirus N proteins. This region has been mapped as the RNA binding domain of the N protein. An intriguing feature of SARS N protein is that it contains five SRXX motifs (Fig. 11A, see highlighted aa sequence); whether that will translate into much higher RNA-binding activity remains to be seen. However, this finding supports the concept of a conserved function within the SR-rich domain.

The appearance of a shorter form of the N-protein late in infection has been observed with TGEV, MHV, FIPV, BCV, avian IBV & TCV coronaviruses (∼2 to 5kDa less) in cell culture. It has been demonstrated that host cell caspases, which are activated during coronavirus infection, are responsible for this cleavage (25). A common caspase cleavage motif is present in all of the mentioned coronavirus N proteins. Furthermore the accumulation of the shortened N protein form was correlated with a 2 log fold reduction in virus production. These observation suggest that cleavage of viral nucleocapsid protein by host cell caspases could be a general mechanism by which infected cells eliminate coronaviruses. Interestingly, no caspase cleavage motif is present in the SARS related coronavirus N-protein.

*The spike protein S -* The spike protein is a major target of the cellular immune response to coronaviruses, and plays an important role in the initial stages of infection. It mediates the attachment of the virus to the cell surface receptors and induces the fusion of the viral and cellular membranes. With several types of viruses, the importance of N-glycosylation on attachment proteins has often been highlighted in virus-receptor interactions.

In influenza C and A viruses, which are myxoviruses, Rosenthal et al. showed that N-glycosylation of the hemagglutinin-esterase-fusion proteins can have dramatic effects on immune escape, virulence, and interactions with cellular receptors (26). The hemagglutinins have been shown to interact with sialic acid moieties on the receptors, and it is known that neuraminidase inhibitors inhibit the replication of influenzaviruses A and B (27)

Hepatitis viruses have been shown to bud into the ER and depend on N-glycosylation of coat proteins to form infectious virus particles (28, 29). Dwek and his colleagues investigated the effect of deoxynojirimycin, an alpha-glucosidase inhibitor, and found that it would block oligosaccharide processing at the stage of monoglucosylation of Asn sites. The glucosylated proteins were shown to misfold. Even at very low inhibitor concentration, viral titers dropped by nearly 100-fold (30). Studies in animals showed that deoxynojirimycin had a negligible effect on host glycosylation (31) and thus drugs such as this alpha-glucosidase inhibitor are seen as good candidates for treatment of hepatitis B. Hepatitis C virus may also respond to these inhibitors (32). Similar studies using sugar inhibitors on HIV, which has many N-linked sites, but showed less sensitivity to misglycosylation (33).

Rossen and co-workers modified the N-glycosylation characteristics of coronavirus spike proteins in cultured epithelial cells, and found that N-glycans had an important impact virus formation and behaviour. For example, inhibition of spike N-glycosylation by tunamycin, which inhibits the synthesis of N-glycans, resulted in the synthesis of spikeless virions, which however still had the capacity of releasing virions (34). The same authors also discussed the implications of N-glycosylation of hemagglutinin proteins of epithelial cell coronaviruses (34).

The SARS-associated coronavirus genome sequence shows that it does not contain a gene encoding hemagglutinin or large genes derived from another virus or host cell (35). It is believed that host range, tissue tropism, and virulence of animal coronaviruses can be changed by mutating the S gene, thus modifying the S proteins (35).

Although no drugs with proven efficacy are currently approved against coronaviruses, potential targets exist for new drugs. For example, protease inhibitors could prevent processing of the RNA polymerase or cleavage of the viral S glycoprotein. Finding antibodies against the S glycoprotein or against the unidentified SARS coronavirus receptor are possible routes to take (35). Also, the use of glycosylation inhibitors which have minimal effects on host cells would be an interesting avenue (30). Very recently, an important contribution by Hilgenfeld et al. (36) outlined a plan for drug design based on inhibition of the viral main proteinase, called M pro or 3CL pro , which controls the activities of the coronavirus replication complex (36).

Ideas for development of vaccines against SARS include the use of killed or subunit vaccines containing the spike glycoprotein with other viral proteins.

*Summation -* The present studies provide the first descriptions of proteins derived from the novel coronavirus thought to be the etiologic agent of SARS. Similar to the pattern observed with animal coronaviruses (21), the 46 kDa nucleoprotein appears to be the major immunogenic antigen, as it was the only viral protein recognised by acute and early convalescent sera from several patients recovering from SARS. While the immune response to the NP could serve as an early diagnostic marker for infection, it is unlikely that an immune response to this protein offers protection, since it is an internal protein and neutralizing antibodies are more likely to target the surface proteins (6). However, it has been shown for other coronaviruses that some antigenic peptides of the N protein can be recognized on the surface of infected cells by T cells (6).

The application of *de novo* sequencing by mass spectrometry provides an alternative to the usual genomic approach for protein identification. Thus it was possible to identify the 46 kDa protein as a novel coronavirus nucleocapsid protein even in the absence of initial genomic sequence. It also provided unequivocal characterization of the proteins produced, including the definition of post-translational modifications. However, perhaps its most important contribution is to distinguish the actual proteins expressed from those that are simply hypothesized or predicted by the nucleotide sequence.

### FULL CITATIONS FOR REFERENCES REFERRED TO IN THE SPECIFICATION

1. Poutanen, S. M. et al. (2003) Identification of severe acute respiratory syndrome in Canada. N Engl. J. Med. 348:1995-2005
2. Booth, C. M. et al, (2003) Clinical features and short-term outcomes of 144 patients with SARS in the greater Toronto area. JAMA May 6, [epub. ahead of print]
3. Marra, M. A. et al, (2003) The genome sequence of the SARS-associated coronavirus. Science May 1, [epub. ahead of print]
4. Rota, P. A. et al, (2003) Characterization of a novel coronavirus associated with severe acute respiratory syndrome. Science May 1, [epub. ahead of print]
5. Thomas, J. J., R. Bakhtiar, R., G. Siuzdak, G. (2001) Mass spectrometry in viral proteomics. Acc. Chem. Res. 33, 179-187
6. Seifers, D. L., Harvey, T. L., Haber, S., She, Y-M., Chernushevich, I., Ens, W., and Standing, K. G. (1999) Natural infection of sorghum by foxtail mosaic disease in Kansas. Plant Disease 83, 905-912
7. Seifers, D. L., Salomon, R., Marie-Jeanne, V., Alliot, B., Signoret, P., Haber, S., Loboda, A., Ens, W., She, Y-M., and Standing, K. G. (2000) Characterization of a novel potyvirus isolated from maize in Israel, Phytopathology 90, 505-513.
8. She, Y-M, Haber, S., Seifers, D. L., Loboda, A., Chernushevich, I., Perreault, H., Ens, W., and Standing, K. G. (2001) Determination of the complete amino acid sequence for the coat protein of brome mosaic virus by time-of -flight mass spectrometry, J. Biol. Chem. 276, 20039-20047.
9. Swanson, M. I., She, Y-M, Ens, W., Brown, E. G., and Coombs, K. M. (2002) Mammalian reovirus core protein micro 2 initiates at the first start codon and is acetylated. Rapid Commun. Mass Spectrom. 16, 2317-2324.
10. Mendez, I. I., She, Y. M., Ens, W., and Coombs, K. M. (2003) Digestion pattern of reovirus outer capsid protein s3 determined by mass spectrometry. Virology, in press.
11. Li, Y., unpublished.
12. Lai, M. M. C., Holmes, K. V. (2001) in Fields Virology (4th ed), pp. 1163-1185.
13. Shevchenko, A., Wilm, M., Vorm, O., and Mann, M. (1996) Mass spectrometric sequencing of proteins from polyacrylamide gels. Anal. Chem. 68, 850-858.
14. Shevchenko, A., Chernushevich, I., Ens, W., Standing, K. G., Thomson, B., Wilm, M., and Mann, M. (1997) Rapid 'de novo' peptide sequencing by a combination of nanoelectrospray, isotopic labeling and a quadrupole/time-of-flight mass spectrometer. Rapid Commun. Mass Spectrom. 11, 1015-1024.
15. Stewart, II, Thomson, T., and Figeys, D. (2001) 180 labeling: a tool for proteomics. Rapid Commun. Mass Spectrom. 15, 2456-2465.
16. Yao, X., Alfonso, C., and Fenselau C. (2003) Dissection of proteolytic 180 labeling: endoprotease catalyzed 160 to 180 exchange of truncated peptide substrates. J. Proteome Res. 2, 147-152.
17. Krokhin, O., Qian, Y., McNabb, J. R., Spicer, V., Standing, K. G., and Ens, W. (2002) An off-line interface between HPLC and orthogonal MALDI TOF. Am. Soc. for Mass Spectrometry, 50 th ASMS Conference on Mass Spectrometry and Allied Topics, Orlando FL USA, 2-6 June: paper WPP 233.
18. Loboda, A. V., Krutchinsky, A. N., Bromirski, M., Ens, W., and Standing, K. G. (2000). A tandem quadrupole/time-of-flight mass spectrometer with a matrix-assisted laser desorption/ionization source: design and performance. Rapid Commun. Mass Spectrom. 14, 1047-1057.
19. Altschul, S. F., Stephen, F., Madden, T. L., Schaeffer, Zhang, J., Miller, W., and Lipman, D. J. (1997). Gapped BLAST and PSI-BLAST: a new generation of protein database search programs. Nucleic Acids Res. 25, 3389-3402.
20. A. Shevchenko, A., Loboda, A., Sunyaev, S., Shevchenko, A., Bork, P., Ens, W., and Standing, K. G. (2001). Charting the proteomes of organisms with unsequenced genomes by MALDI-quadrupole time-of-flight mass spectrometry and BLAST homology searching. Anal. Chem. 73, 1917-1926.
21. NetNglyc 1.0 server: http//www.cbs.dtu.dk/services/NetNGlyc/
22. Hogue, B. G., and Brian, D. A. (1987). Glycosylation of the bovine coronavirus hemagglutinin protein. Adv. Exp. Med. Biol. 218, 131-136
23. Thompson, J. D., Higgins, D. G., Gibson, T. J. (1994). CLUSTAL W: improving the sensitivity of progressive multiple sequence alignment through sequence weighting, position-specific gap penalties, and weight matrix choice. Nucleic Acids Res. 22, 4673-4680.
24. Nelson, G. W., Stohlman, S.A., Tahara, S.M. (2000). High affinity interaction between nucleocapsid protein and leader/intergenic sequence of mouse hepatitis virus RNA. J Gen Virol. 81,181-188.
25. Eleouet, J. F., Slee, E. A., Saurini, F., Castagne, N., Poncet, D., Garrido, C., Solary, E, and Martin S. J. (2000). The viral nucleocapsid protein of transmissible gastroenteritis coronavirus (TGEV) is cleaved by caspase-6 and -7 during TGEV-induced apoptosis. J Virol. 74, 3975-3983.
26. Rosenthal, P.B., Zhang, X., Formanowski, F., Fitz, W., Wong, C. H., Meier-Ewert, H., Skehel, J. J., and Wiley, D. C. (1998) Structure of the haemagglutinin-esterase-fusion glycoprotein of influenza C virus. Nature 396 92-96.
27. Oxford, J. S., Bossuyt, S., Balasingam, S., Mann, A., Novelli, P., Lambkin, R. (2003). Clin Microbiol Infect. 9, 1-14.
28. Lu, X., Mehta, A., Dwek, R., Butters, T., Block, T. (1995). Evidence that N-linked glycosylation is necessary for hepatitis B virus secretion. Virology 213, 660-665.
29. Mehta, A., Block, T. M., Dwek, R. A. (1998). The role of N-glycosylation in the secretion of hepatitis B virus. Adv. Exp. Med. Biol. 435, 195-205.
30. Mehta, A., Rudd, P. M., Block, T. M., Dwek, R. A. (1997). A strategy for anti-viral intervention: the use of alpha-glycosidase inhibitors to prevent chaperone-mediated folding of viral envelope glycoproteins. Biochem. Soc. Trans. 25, 1188-1193.
31. Freeze, H. H., Westphal, V. C. (2001). Balancing N-linked glycosylation to avoid disease. Biochimie 83, 791-799.
32. Ziztzmann, N., Mehta, A. S., Carrouee, S., Butters, T. D., Platt, F. M., McCauley, J., Blumberg, B. S., Dwek, R. A., Block, T. M. (1999). Imino sugars inhibit the formation and secretion of bovine diarrhea virus, a pestivirus model of hepetitis C virus: implications for the development of broad spectrum anti-hepatitis virus agents. Proc. Natl. Acad. Sci. USA 96, 11878-11882.
33. Mehta, A., Zitzmann, N., Rudd, P. M., Block, T. M., Dwek, R. A. (1998). Alpha-glucosidase inhibitors as potential broad-based anti-viral agents. FEBS Lett. 430, 17-22.
34. Rossen, J. W., de Beer, R., Godeke, G. J., Raamsman, M. J., Horzinek, M.C., Vennema, H., and Rottier, P. J. (1998) The viral spike protein is not involved in the polarized sorting of coronaviruses in epithelial cells. J Virol. 72, 497-503.
35. Peiris, J. S. M., Lai, S. T., Poon, L. L. M., Guan, Y., Yam, L. Y. C., Lim, W., Nicholls, J., Yee, W. K. S., Yan, W. W., Cheung, M. T., Cheng, V. C. C., Chan, K. H., Tsang, D. N. C., Yung, R. W. H., Ng, T. N., Yuen, K. Y., SARS study group (2003).Coronavirus as a possible cause of severe acute respiratory syndrome. Lancet 361,1319-1325.
36. Anand, K., Ziebuhr, J., Wadhwani, P., Mesters, J. R., Hilgenfeld, R. (2003). Coronavirus Main Proteinase (3CL pro ) Structure: Basis for Design of Anti-SARS Drugs. Science, Published online 13 May, 10.1126/science.1085658.

**Table 1 Calculated and measured masses for b and y ions from MS/MS measurements of the 2297.092 Da tryptic fragment.**

| **y-ion** | ***m*/*z* found** | ***m*/*z* calc.** | **△m (mDa)** | **Residue** | **△m (mDa)** | ***m*/*z* found** | ***m*/*z* calc.** | **b-ion** |
|---|---|---|---|---|---|---|---|---|
| y1 | - | 147.113 | - | **K** | 2 | 2279.083 | 2279.081 | b20 |
| y2 | 261.138 | 261.156 | -18 | **N** | - | - | 2150.986 | b19 |
| y3 | 332.193 | 332.193 | | **A** | | | 2036.943 | b18 |
| y4 | 389.197 | 389.215 | -18 | **G** | 58 | | 1965.906 | b17 |
| y5 | 552.283 | 552.278 | 5 | **Y** | 0 | 1908.885 | 1908.885 | b16 |
| y6 | 649.328 | 649.331 | -3 | **P** | -13 | 1745.809 | 1745.822 | b15 |
| y7 | 762.413 | 762.415 | -2 | **L** | 21 | 1648.790 | 1648.769 | b14 |
| y8 | 849.438 | 849.447 | -9 | **S** | 8 | 1535.694 | 1535.685 | b13 |
| y9 | 920.478 | 920.484 | -6 | **A** | -15 | 1448.638 | 1448.653 | b12 |
| y10 | 1049.522 | 1049.526 | -4 | **E** | 19 | 1377.637 | 1377.616 | b11 |
| y11 | 1146.571 | 1146.579 | -8 | **P** | - | - | 1248.573 | b10 |
| y12 | 1203.592 | 1203.601 | -9 | **G** | -4 | 1151.516 | 1151.520 | b9 |
| y13 | 1304.636 | 1304.648 | -8 | **T** | -5 | 1094.494 | 1094.499 | b8 |
| y14 | 1361.654 | 1361.670 | -16 | **G** | 1 | 993.452 | 993.451 | b7 |
| y15 | 1474.739 | 1474.754 | -15 | **L** | -21 | 936.409 | 936.430 | b6 |
| y16 | 1637.818 | 1637.817 | 1 | **Y** | 11 | 823.357 | 823.346 | b5 |
| y17 | 1800.884 | 1800.881 | 3 | **Y** | 9 | 660.291 | 660.282 | b4 |
| y18 | 1947.970 | 1947.949 | 21 | **F** | 1 | 497.220 | 497.219 | b3 |
| y19 | 2111.022 | 2111.012 | 10 | **Y** | -5 | 350.145 | 350.150 | b2 |
| y20 | 2297.086 | 2297.092 | -6 | **W** | - | - | 187.087 | b1 |

**Table 2 Measured and calculated m/z values of peptides found in tryptic digest of the 46-kDa protein. M* represents oxidized methionine residues: Ac-SDN... is the acetylated N-terminal of the protein: Q** represents the N-terminal Gln residues converted into pyro-Glu; N*** represents an Asn residue converted into Asp due to deamidation.**

| ***m*/*z* measured** | **MH⁻ calculated** (Da) | △**m (mDa)** | **Residues start-end** | **Peptide sequence** |
|---|---|---|---|---|
| 601.303 | 601.305 | -3 | 204-209 | GNSPAR |
| 601.322 | 601.331 | 1 | 103-107 | ELSPR |
| 698.357 | 698.358 | -1 | 144-149 | DHIGTR |
| 708.330 | 708.335 | -5 | 96-102 | GGDGKM*K |
| 711.333 | 711.331 | 2 | 294-299 | QGTDYK |
| 746.387 | 746.383 | 4 | 356-361 | HIDAYK |
| 749.354 | 749.354 | 0 | 178-185 | GGSQASSR |
| 805.378 | 805.380 | -2 | 196-203 | NSTPGSSR |
| 831.462 | 831.457 | 5 | 227-233 | LNQLESK |
| 876.452 | 876.461 | -9 | 101-107 | M*KELSPR |
| 886.401 | 886.406 | -5 | 170-177 | GFYAEGSR |
| 916.478 | 916.478 | 0 | 362-369 | TFPPTEPK |
| 928.543 | 928.546 | -3 | 348-355 | DNVILLNK |
| 946.513 | 946.511 | 2 | 62-68 | EELRFPR |
| 1105.548 | 1105.553 | -5 | 339-347 | LDDKDPQFK |
| 1144.493 | 1144.499 | -6 | 1-10 | Ac-SDNGPQSNC1R |
| 1154.579 | 1154.580 | -1 | 376-387 | TDEAQPLPQR |
| 1166.557 | 1166.559 | -2 | 267-276 | Q**YNVTQAFGR |
| 1183.589 | 1183.586 | 3 | 267-276 | QYNVTQAFGR |
| 1202.610 | 1202.613 | -3 | 238-248 | GQQQQGQTVTK |
| 1282.678 | 1282.675 | 3 | 375-385 | KTDEAQPLPQR |
| 1330.698 | 1330.708 | -10 | 238-249 | GQQQQGQTVTKK |
| 1410.774 | 1410.771 | 3 | 376-387 | KKTDEAQPLPQR |
| 1611.698 | 1611.692 | 6 | 406-421 | QLQNSM*SGASADSTQA |
| 1684.895 | 1684.891 | 4 | 128-293 | EGIVWVATEGALNTPK |
| 1687.898 | 1687.905 | -7 | 210-226 | MASGGGETALALLLLDR |
| 1703.897 | 1703.900 | -3 | 210-226 | M*ASGGGETALALLLLDR |
| 1774.838 | 1774.836 | 2 | 278-293 | GPEQTQGNFGDQDLIR |
| 1850.833 | 1850.827 | 6 | 15-32 | ITFGGPTDSTDNNONGGR |
| 1851.814 | 1850.811 | 3 | 15-32 | ITFGGPTDSTDNNQN***GGR |
| 1875.879 | 1875.879 | 0 | 389-405 | Q**PTVTLLPAADM*DDFSR |
| 1892.905 | 1892.906 | -1 | 389-405 | QPTVTLLPAADM*DDFSR |
| 1930.944 | 1930.937 | 7 | 277-293 | RGPEQTQGNFGDQDLIR |
| 2005.008 | 2005.006 | 2 | 388-405 | KQPTVTLLPAADMDDFSR |
| 2015.081 | 2015.081 | 0 | 339-355 | LDDKDPQFKDNVILLNK |
| 2021.005 | 2021.001 | 4 | 388-405 | KQPTVTLLPAADM*DDFSR |
| 2077.048 | 2077.043 | 5 | 320-338 | IGM*EVTPSGTWLTYHGAIK |
| 2091.126 | 2091.120 | -2 | 150-169 | NPNNNAATVLQLPQGTTLPK |
| 2151.002 | 2151.010 | 6 | 69-88 | GQGVPINTNSGPDDQIGYYR |
| 2252.062 | 2252.071 | -9 | 300-319 | HWPQIAQFAPSASAFFGMSR |
| 2297.078 | 2297.092 | -14 | 108-127 | WYFYYLGTGPEASLPYGANK |
| 2307.128 | 2307.111 | 17 | 69-89 | GQGVPINTNSGPDDQIGYYRR |
| 2324.187 | 2324.190 | -3 | 41-61 | RPQGLPNNTASWFTALTQHGK |
| 2516.325 | 2516.339 | -14 | 210-233 | M*ASGGGETALALLLLDRLNQLESK |

**Table 3 Deglycosylated peptides found in PNGase F-treated tryptic digest of 139-kDa spike protein. Bold N symbols represented deglycosylated sites.**

| **Peptide** | **Sequence*** | **Calc. [M+H]+ with no glycosylation** | **[M+H]+ measured after PNGase** | ***m*/*z* diff.** | **No. sites** | **MS/MS deglyco. Peptide** |
|---|---|---|---|---|---|---|
| T111-126 | SQSVIIINNSTNVVIR | 1756.992 | 1757.973 | 0.981 | 1 | X |
| | SQSVIIINNSTNVVIR | | 1758.963 | 1.971 | 2 | X |
| T222-232 | LPLGINITNFR | 1257.732 | 1258.715 | 0.983 | 1 | X |
| T226-287 | YDENGTITDAV | 2453.114 | 2454.101 | 0.987 | 1 | X |
| | DCSQNPLAELK | | | | | |
| T316-333 | FPNITNLCFP GEVFNATK | 2069.017 | 2071.001 | 1.984 | 2 | X |
| T778-796 | YFGGFNFSQ ILPDPLKPTK | 2169.138 | 2170.121 | 0.983 | 1 | X |
| T1056-1068 | NFITAPAICHEGK | 1445.685 | 1446.672 | 0.987 | 1 | X |
| T1074-1089 | EGVFVFNG TSWFITQR | 1887.939 | 1888.931 | 0.922 | 1 | X |
| T1140-1163 | NHTSPDVDLGDI | 2493.259 | 2495.23 | 1.971 | 2 | X |
| | SGINASVVNIQK | | | | | |
| T1174-1187 | NLNESLIDLQELGK | 1585.844 | 1586.823 | 0.979 | 1 | X |

**Table 4 Glycosylated peptides found in tryptic digest of 139-kDa spike protein without treatment with PNGase F.**

| **Peptide** | **Sequence** | **Calc. [M+H]+ *m*/*z*** | **[M+H]+, meas. glyco-peptide** | **Risidual Carbo. mass** | **Carbohydr Composition** | **Calc. carbo. mass** | **mass (meas. -calc.)** | **MS/MS glyco-peptide** |
|---|---|---|---|---|---|---|---|---|
| T111-126 | SQSVIIIN | 1756.992 | 3201.514 | 1444.522 | M3N4F | 1444.534 | -0.012 | |
| | NSTNVV | | 3404.572 | 1647.580 | M3N5F | 1647.613 | -0.033 | X |
| | IR | | 3525.669 | 1768.677 | M3N4G2F | 1768.640 | 0.037 | |
| | | | 3566.670 | 1809.678 | M3N5GF | 1809.666 | 0.012 | |
| | | | 3607.682 | 1850.690 | M3N6F | 1850.693 | -0.003 | X |
| | | | 3728.687 | 1971.695 | M3N5G2F | 1971.719 | -0.024 | |
| | | | 3769.678 | 2012.686 | M3N6GF | 2012.745 | -0.059 | |
| | | | 3890.761 | 2133.769 | M3N5G3F | 2133.772 | -0.003 | |
| | | | 3931.793 | 2174.801 | M3N6G2F | 2174.798 | 0.030 | |
| | | | 4093.818 | 2336.826 | M3N6G3F | 2336.859 | -0.025 | X |
| | | | 4255.844 | 2498.852 | M3N6G4F | 2498.904 | 0.052 | |
| | | | 4296.905 | 2539.913 | M3N7G3F | 2539.930 | -0.017 | |
| | | | | | | | | |
| T222-232 | LPLGINI | 1257.732 | 2636.215 | 1378.483 | M6N2 | 1378.746 | 0.007 | |
| | TNFR | | 2798.26 | 1540.528 | M7N2 | 1540.528 | 0.000 | |
| | | | 2960.306 | 1702.574 | M8N2 | 1702.574 | -0.007 | |
| | | | 3122.373 | 1864.641 | M9N2 | 1864.641 | 0.007 | X |
| | | | | | | | | |
| T226-287 | YDENGT | 2453.114 | 4100.666 | 1647.552 | M3N5F | 1647.613 | -0.061 | |
| | ITDAVD | | 4303.735 | 1850.621 | M3N6F | 1850.693 | 0.072 | X |
| | CSQNPL | | 4427.1* | 1974.0 | M3N5FG2 | 1971.719 | | |
| | AELK | | 4468.9* | 2015.8 | M3N6FG | 2012.745 | | |
| | | | 4589.1* | 2136.0 | M3N5G3F | 2133.772 | | |
| | | | 4792.2* | 2399.1 | M3N6G3F | 2336.859 | | |
| | | | 4953.9* | 2500.8 | M3N6G4F | 2498.904 | | |
| T778-796 | YFGGFN | 2169.138 | 3385.59 | 1216.452 | M5N2 | 1216.423 | -0.029 | |
| | FSQILPD | | 3547.644 | 1378.506 | M6N2 | 1378.476 | -0.030 | |
| | PLKPTK | | 3709.738 | 1540.600 | M7N2 | 1540.529 | -0.071 | X |
| | | | 3871.702 | 1702.564 | M8N2 | 1702.581 | 0.017 | |

| | | | | | | | | |
|---|---|---|---|---|---|---|---|---|
| *Peptide Symbols* **N:** first glycosylation site **N:** second glycoylation site *Carbohydrate composition symbols:* **M** = mannose **F** = fucose **G** = galactose **N** = N-acctyl glucosamine **m*/*z* values could not be determined accurately (see text) | | | | | | | | |

**Table 5 Measured and calculated m/z values of peptides found in tryptic digests of the 139-kDas protein. Mass values are taken directly from the automatic peak finding program, and are therefore somewhat less reliable than when they are checked manually. A) without deglycosylation of the 39 peptides observed (34% coverage). 36 were confirmed by MS/MS (30% coverage). B) After deglycosylation with PNGaseF (see text) nine deglycosylated peptides were found and confirmed by MS/MS giving an additional 12% coverage. M* - oxidized Met residues; **-peptide identifications not confirmed by MS/MS: D - deglycosylated Asn residues.**

| | ***m*/*z* measured** | **MH-Calculated (Da)** | Δ**m (mDa)** | **Residues start-end** | **Peptide sequence** |
|---|---|---|---|---|---|
| **A** | | | | | |
| | 653.344 | 653.341 | 3 | 1069-1073 | AYFPR |
| | 669.361 | 669.361 | 0 | 184-188 | EFVFK |
| | 673.389 | 673.388 | 1 | 1168-1173 | LNEVAK** |
| | 725.332 | 725.329 | 3 | 1022-1027 | VDFCGK |
| | 738.370 | 738.367 | 3 | 292-297 | SFEIDK |
| | 817.478 | 817.468 | 10 | 448-453 | LRPFER |
| | 820.470 | 820.457 | 13 | 762-768 | EVFAQVK |
| | 846.476 | 846.468 | 8 | 1002-1010 | ASANLAATK |
| | 847.453 | 847.434 | 19 | 830-836 | DLICAQK |
| | 881.446 | 881.430 | 16 | 1021-1027 | RVDFCGK |
| | 885.420 | 885.414 | 6 | 366-373 | CYGVSATK |
| | 927.543 | 927.526 | 17 | 307-315 | VVPSGDVVR |
| | 962.523 | 962.506 | 17 | 904-911 | QIANQFNK |
| | 1046.556 | 1046.563 | -7 | 199-207 | GYQPIDVVR |
| | 1068.533 | 1068.540 | -7 | 808-817 | VTLADAGFM*K** |
| | 1085.553 | 1085.538 | 15 | 298-306 | GIYQTSNFR |
| | 1114.534 | 1114.542 | -8 | 85-94 | DGIYFAATEK |
| | 1130.592 | 1130.581 | 11 | 748-758 | ALSQIAAEQDR |
| | 1154.555 | 1154.563 | -8 | 334-342 | FPSVYAWER |
| | 1154.564 | 1154.574 | -10 | 545-553 | FOFPQQFGR |
| | 1225.641 | 1225.647 | -6 | 798-807 | SFIEDLLFNK |
| | 1246.646 | 1246.647 | -1 | 189-198 | NKDGFLYVYK |
| | 1258.602 | 1258.610 | -8 | 39-48 | GVYYFDEIFR |
| | 1310.663 | 1310.675 | -12 | 544-553 | RFOPFQQFQR |
| | 1395.622 | 1395.632 | -10 | 818-829 | QYGECLGDINAR |
| | 1414.754 | 1414.754 | 0 | 966-977 | LDKVEAEVQIDR |
| | 1460.690 | 1460.666 | 24 | 427-439 | NIDATSTGNYNYK |
| | 1480.687 | 1480.692 | -5 | 554-566 | DVSDFTDSVRDPK |
| | 1575.866 | 1576.874 | -8 | 208-221 | DLPSQFNTLKPIFK |
| | 1690.951 | 1690.949 | 2 | 983-996 | LQSLQTYVTQQLIR |
| | 1737.879 | 1737.881 | -2 | 396-411 | QIAPGQTGVIADYNYK |
| | 1804.885 | 1804.887 | -2 | 292-306 | SFEIDKGIYQTSNFR |
| | 1810.834 | 1810.825 | -1 | 412-436 | LPDDFM*QCVLAWNTR |
| | 1848.979 | 1848.992 | -13 | 912-929 | AISQIQESLTTTSTALGK |
| | 1868.012 | 1868.024 | -12 | 930-946 | LQDVVNQNAQALNTLVK |
| | 1990.954 | 1990.958 | -4 | 374-390 | LNDLCFSNVYADSFVVK |
| | 2014.091 | 2014.104 | -13 | 496-514 | VVVLSFELLNAPATVCGPK |
| | 2355.139 | 2355.140 | -1 | 522-543 | NQCVNFNFNGLTGTGVLTPSSK |
| | 3183.578 | 3183.584 | -6 | 1028-1055 | GYHLM*SFPQAAPHGVVFLHVTYVPSOER** |
| | | | | | |

| **B Deglycosylated peptides found in PNGase-treated tryptic digest** | | | | | |
|---|---|---|---|---|---|
| | 1258.715 | 1258.716 | -1 | 222-232 | LPLGIDITNFR |
| | 1446.672 | 1446.669 | 3 | 1056-1068 | DFTTAPAICHEGK |
| | 1586.803 | 1586.808 | -5 | 1174-1187 | NLDESLIDLQELGK |
| | 1757.973 | 1757.976 | -3 | 111-126 | SQSVIIINDSTNVVIR |
| | 1758.963 | 1758.960 | 3 | 111-126 | SQSVIIIDDSTNVVIR |
| | 1888.931 | 1888.923 | 8 | 1074-1089 | EGVFVFDGTSWFITOR |
| | 2071.001 | 2070.985 | 16 | 316-333 | FPDITNLCPFGEVFDATK |
| | 2170.121 | 2170.122 | -1 | 778-796 | YEGGFDFSOILPDPLKPTK |
| | 2454.101 | 2454.098 | 3 | 226-287 | YDEDGTITDAVDCSONFLAELK |
| | 2495.230 | 3495.227 | 3 | 1140-1163 | DHTSPDVDLGDISGIDASVVNIQK |

## Claims

1. An isolated SARS-related protein having a sequence as set forth in SEQ ID No. 1.

2. An antibody reactive against a protein according to claim 1.

3. A method of detecting a coronavirus or a condition associated with a coronavirus comprising assaying a sample for (a) the SARS-related protein according to claim 1 or a immunogenic fragment thereof; or (b) an antibody that binds the SARS-related protein according to claim 1.

4. A method according to claim 3 which comprises contacting a SARS-related protein of claim 1 or an immunogenic fragment thereof, with the sample and determining the presence of antibodies to the SARS-related protein in the sample.

5. A method according to claim 3 which comprises contacting the antibody of claim 2 with the sample and determining the presence of a SARS-related protein in the sample.

6. A method according to any one of claims 3 to 5 wherein the condition is SARS.

7. A reagent comprising an amount of (a) a SARS-related protein according to claim 1 or an immunogenic fragment thereof; or (b) an antibody that binds a SARS-related protein according to claim 1 in admixture with a suitable diluent or carrier.

8. A method of screening for a compound capable of binding to a protein according to claim 1 or a immunogenic fragment thereof comprising contacting the protein or fragment thereof with a test compound and determining the ability of said test compound to bind to said protein or fragment thereof.

## Patentansprüche

1. Isoliertes SARS-verwandtes Protein mit einer in SEQ ID Nr. 1 dargestellten Sequenz.

2. Antikörper mit Reaktivität gegenüber einem Protein nach Anspruch 1.

3. Verfahren zum Nachweis eines Coronavirus oder eines mit einem Coronavirus assoziierten Zustands umfassend das Untersuchen einer Probe auf (a) das SARS-verwandte Protein nach Anspruch 1 oder ein immunogenes Fragment desselben oder auf (b) einen Antikörper, der an das SARS-verwandte Protein nach Anspruch 1 bindet.

4. Verfahren nach Anspruch 3, welches das Kontaktieren eines SARS-verwandten Proteins nach Anspruch 1 oder eines immunogenen Fragments desselben mit der Probe und das Bestimmen des Vorhandenseins von Antikörpern gegen das SARS-verwandte Protein in der Probe umfasst.

5. Verfahren nach Anspruch 3, welches das Kontaktieren des Antikörpers nach Anspruch 2 mit der Probe und das Bestimmen des Vorhandenseins eines SARS-verwandten Proteins in der Probe umfasst.

6. Verfahren nach einem der Ansprüche 3 bis 5, wobei es sich bei dem Zustand um SARS (schweres akutes Atemwegssyndrom) handelt.

7. Reagenz umfassend eine Menge (a) eines SARS-verwandten Proteins nach Anspruch 1 oder eines immunogenen Fragments desselben oder (b) eines Antikörpers, der an ein SARS-verwandtes Protein nach Anspruch 1 bindet, in einer Mischung mit einem geeigneten Verdünnungsmittel oder Träger.

8. Verfahren zur Screeningprüfung auf eine Verbindung mit der Fähigkeit, an ein Protein nach Anspruch 1 oder ein immunogenes Fragment desselben zu binden, umfassend das Kontaktieren des Proteins oder Fragments desselben mit einer Prüfverbindung und das Bestimmen der Fähigkeit der Prüfverbindung zur Bindung an das Protein oder Fragment desselben.

## Revendications

1. Protéine isolée associée au SRAS présentant une séquence telle que définie dans la SEQ ID N°1.

2. Anticorps réagissant contre une protéine selon la revendication 1.

3. Procédé de détection d'un coronavirus ou d'un état associée à un coronavirus comprenant une analyse d'un échantillon pour (a) la protéine associée au SRAS selon la revendication 1 ou un fragment immunogène de celle-ci ; ou (b) un anticorps qui se lie à la protéine associée au SRAS selon la revendication 1.

4. Procédé selon la revendication 3 qui comprend la mise en contact d'une protéine associée au SRAS selon la revendication 1 ou d'un fragment immunogène de celle-ci avec l'échantillon et la détermination de la présence d'anticorps contre la protéine associée au SRAS dans l'échantillon.

5. Procédé selon la revendication 3 qui comprend la mise en contact de l'anticorps selon la revendication 2 avec l'échantillon et la détermination de la présence d'une protéine associée au SRAS dans l'échantillon.

6. Procédé selon l'une quelconque des revendications 3 à 5, l'état étant le SRAS.

7. Réactif comprenant une quantité (a) d'une protéine associée au SRAS selon la revendication 1 ou d'un fragment immunogène de celle-ci ; ou (b) d'un anticorps qui se lie à une protéine associée au SRAS selon la revendication 1 dans un mélange comprenant un diluant ou un vecteur approprié.

8. Procédé de dépistage (screening) d'un composé capable de se lier à une protéine selon la revendication 1 ou d'un fragment immunogène de celle-ci comprenant la mise en contact de la protéine ou d'un fragment de celle-ci avec un composé test et la détermination de la capacité dudit composé test à se lier à ladite protéine ou au dit fragment de celle-ci.


### SEQUENCE LISTING

<110> University of Manitoba Her Majesty the Queen in Right of Canada as Represented by the Minister of Health
<120> SARS-RELATED PROTEINS
<130> 85084-1003
<150> US 60/462688
   <151> 2003-04-15
<150> US 60/472416
   <151> 2003-05-22
<160> 2
<170> PatentIn version 3.2
<210> 1
   <211> 421
   <212> PRT
   <213> coronavirus
<400> 1
   Ser Asp Asn Gly Pro Gln Ser Asn Gln Arg Ser Ala Pro Arg Ile Thr
   1               5                   10                  15
   Phe Gly Gly Pro Thr Asp Ser Thr Asp Asn Asn Gln Asp Gly Gly Arg
               20                  25                  30
   Asn Gly Ala Arg Pro Lys Gln Arg Arg Pro Gln Gly Leu Pro Asn Asn
           35                  40                  45
   Thr Ala Ser Trp Phe Thr Ala Leu Thr Gln His Gly Lys Glu Glu Leu
       50                  55                  60
   Arg Phe Pro Arg Gly Gln Gly Val Pro Ile Asn Thr Asn Ser Gly Pro
   65                  70                  75                  80
   Asp Asp Gln Ile Gly Tyr Tyr Arg Arg Ala Thr Arg Arg Val Arg Gly
                   85                  90                  95
   Gly Asp Gly Lys Met Lys Glu Leu Ser Pro Arg Trp Tyr Phe Tyr Tyr
               100                 105                 110
   Leu Gly Thr Gly Pro Glu Ala Ser Leu Pro Tyr Gly Ala Asn Lys Glu
           115                 120                 125
   Gly Ile Val Trp Val Ala Thr Glu Gly Ala Leu Asn Thr Pro Lys Asp
       130                 135                 140
   His Ile Gly Thr Arg Asn Pro Asn Asn Asn Ala Ala Thr Val Leu Gln
   145 150 155 160
   Leu Pro Gln Gly Thr Thr Leu Pro Lys Gly Phe Tyr Ala Glu Gly Ser
   165 170 175
   Arg Gly Gly Ser Gln Ala Ser Ser Arg Ser Ser Ser Arg Ser Arg Gly
   180 185 190
   Asn Ser Arg Asn Ser Thr Pro Gly Ser Ser Arg Gly Asn Ser Pro Ala
   195 200 205
   Arg Met Ala Ser Gly Gly Gly Glu Thr Ala Leu Ala Leu Leu Leu Leu
   210 215 220
   Asp Arg Leu Asn Gln Leu Glu Ser Lys Val Ser Gly Lys Gly Gln Gln
   225 230 235 240
   Gln Gln Gly Gln Thr Val Thr Lys Lys Ser Ala Ala Glu Ala Ser Lys
   245 250 255
   Lys Pro Arg Gln Lys Arg Thr Ala Thr Lys Gln Tyr Asn Val Thr Gln
   260 265 270
   Ala Phe Gly Arg Arg Gly Pro Glu Gln Thr Gln Gly Asn Phe Gly Asp
   275 280 285
   Gln Asp Leu Ile Arg Gln Gly Thr Asp Tyr Lys His Trp Pro Gln Ile
   290 295 300
   Ala Gln Phe Ala Pro Ser Ala Ser Ala Phe Phe Gly Met Ser Arg Ile
   305 310 315 320
   Gly Met Glu Val Thr Pro Ser Gly Thr Trp Leu Thr Tyr His Gly Ala
   325 330 335
   Ile Lys Leu Asp Asp Lys Asp Pro Gln Phe Lys Asp Asn Val Ile Leu
   340 345 350
   Leu Asn Lys His Ile Asp Ala Tyr Lys Thr Phe Pro Pro Thr Glu Pro
   355 360 365
   Lys Lys Asp Lys Lys Lys Lys Thr Asp Glu Ala Gln Pro Leu Pro Gln
   370 375 380
   Arg Gln Lys Lys Gln Pro Thr Val Thr Leu Leu Pro Ala Ala Asp Met
   385 390 395 400
   Asp Asp Phe Ser Arg Gln Leu Gln Asn Ser Met Ser Gly Ala Ser Ala
   405 410 415
   Asp Ser Thr Gln Ala
   420
<210> 2
   <211> 1254
   <212> PRT
   <213> coronavirus
<300>
   <301> Krokhin et al. <302> Mass Spectometric characterization of Proteins from the SARS virus: A Preliminary Report
   <303> Mol. Cell. Proteomics
   <304> 2
   <305> 5
   <306> 346-356
   <307> 2003-05-29
<400> 2
   Phe Ile Phe Leu Leu Phe Leu Thr Leu Thr Ser Gly Ser Asp Leu Asp
   1 5 10 15
   Arg Cys Thr Thr Phe Asp Asp Val Gln Ala Pro Asn Tyr Thr Gln His
   20 25 30
   Thr Ser Ser Met Arg Gly Val Tyr Tyr Pro Asp Glu Ile Phe Arg Ser
   35 40 45
   Asp Thr Leu Tyr Leu Thr Gln Asp Leu Phe Leu Pro Phe Tyr Ser Asn
   50 55 60
   Val Thr Gly Phe His Thr Ile Asn His Thr Phe Gly Asn Pro Val Ile
   65 70 75 80
   Pro Phe Lys Asp Gly Ile Tyr Phe Ala Ala Thr Glu Lys Ser Asn Val
   85 90 95
   Val Arg Gly Trp Val Phe Gly Ser Thr Met Asn Asn Lys Ser Gln Ser
   100 105 110
   Val Ile Ile Ile Asn Asn Ser Thr Asn Val Val Ile Arg Ala Cys Asn
   115 120 125
   Phe Glu Leu Cys Asp Asn Pro Phe Phe Ala Val Ser Lys Pro Met Gly
   130 135 140
   Thr Gln Thr His Thr Met Ile Phe Asp Asn Ala Phe Asn Cys Thr Phe
   145                 150                 155                 160
   Glu Tyr Ile Ser Asp Ala Phe Ser Leu Asp Val Ser Glu Lys Ser Gly
                   165                 170                 175
   Asn Phe Lys His Leu Arg Glu Phe Val Phe Lys Asn Lys Asp Gly Phe
               180                 185                 190
   Leu Tyr Val Tyr Lys Gly Tyr Gln Pro Ile Asp Val Val Arg Asp Leu
           195                 200                 205
   Pro Ser Gly Phe Asn Thr Leu Lys Pro Ile Phe Lys Leu Pro Leu Gly
       210                 215                 220
   Ile Asn Ile Thr Asn Phe Arg Ala Ile Leu Thr Ala Phe Ser Pro Ala
   225                 230                 235                 240
   Gln Asp Ile Trp Gly Thr Ser Ala Ala Ala Tyr Phe Val Gly Tyr Leu
                   245                 250                 255
   Lys Pro Thr Thr Phe Met Leu Lys Tyr Asp Glu Asn Gly Thr Ile Thr
               260                 265                 270
   Asp Ala Val Asp Cys Ser Gln Asn Pro Leu Ala Glu Leu Lys Cys Ser
           275                 280                 285
   Val Lys Ser Phe Glu Ile Asp Lys Gly Ile Tyr Gln Thr Ser Asn Phe
       290                 295                 300
   Arg Val Val Pro Ser Gly Asp Val Val Arg Phe Pro Asn Ile Thr Asn
   305                 310                 315                 320
   Leu Cys Pro Phe Gly Glu Val Phe Asn Ala Thr Lys Phe Pro Ser Val
                   325                 330                 335
   Tyr Ala Trp Glu Arg Lys Lys Ile Ser Asn Cys Val Ala Asp Tyr Ser
               340                 345                 350
   Val Leu Tyr Asn Ser Thr Phe Phe Ser Thr Phe Lys Cys Tyr Gly Val
           355                 360                 365
   Ser Ala Thr Lys Leu Asn Asp Leu Cys Phe Ser Asn Val Tyr Ala Asp
       370                 375                 380
   Ser Phe Val Val Lys Gly Asp Asp Val Arg Gln Ile Ala Pro Gly Gln
   385                 390                 395                 400
   Thr Gly Val Ile Ala Asp Tyr Asn Tyr Lys Leu Pro Asp Asp Phe Met
                   405                 410                 415
   Gly Cys Val Leu Ala Trp Asn Thr Arg Asn Ile Asp Ala Thr Ser Thr
               420                 425                 430
   Gly Asn Tyr Asn Tyr Lys Tyr Arg Tyr Leu Arg His Gly Lys Leu Arg
           435                 440                 445
   Pro Phe Glu Arg Asp Ile Ser Asn Val Pro Phe Ser Pro Asp Gly Lys
       450                 455                 460
   Pro Cys Thr Pro Pro Ala Leu Asn Cys Tyr Trp Pro Leu Asn Asp Tyr
   465                 470                 475                 480
   Gly Phe Tyr Thr Thr Thr Gly Ile Gly Tyr Gln Pro Tyr Arg Val Val
                   485                 490                 495
   Val Leu Ser Phe Glu Leu Leu Asn Ala Pro Ala Thr Val Cys Gly Pro
               500                 505                 510
   Lys Leu Ser Thr Asp Leu Ile Lys Asn Gln Cys Val Asn Phe Asn Phe
           515                 520                 525
   Asn Gly Leu Thr Gly Thr Gly Val Leu Thr Pro Ser Ser Lys Arg Phe
       530                 535                 540
   Gln Pro Phe Gln Gln Phe Gly Arg Asp Val Ser Asp Phe Thr Asp Ser
   545                 550                 555                 560
   Val Arg Asp Pro Lys Thr Ser Glu Ile Leu Asp Ile Ser Pro Cys Ala
                   565                 570                 575
   Phe Gly Gly Val Ser Val Ile Thr Pro Gly Thr Asn Ala Ser Ser Glu
               580                 585                 590
   Val Ala Val Leu Tyr Gln Asp Val Asn Cys Thr Asp Val Ser Thr Ala
           595                 600                 605
   Ile His Ala Asp Gln Leu Thr Pro Ala Trp Arg Ile Tyr Ser Thr Gly
       610                 615                 620
   Asn Asn Val Phe Gln Thr Gln Ala Gly Cys Leu Ile Gly Ala Glu His
   625                 630                 635                 640
   Val Asp Thr Ser Tyr Glu Cys Asp Ile Pro Ile Gly Ala Gly Ile Cys
                   645                 650                 655
   Ala Ser Tyr His Thr Val Ser Leu Leu Arg Ser Thr Ser Gln Lys Ser
   660 665 670
   Ile Val Ala Tyr Thr Met Ser Leu Gly Ala Asp Ser Ser Ile Ala Tyr
   675 680 685
   Ser Asn Asn Thr Ile Ala Ile Pro Thr Asn Phe Ser Ile Ser Ile Thr
   690 695 700
   Thr Glu Val Met Pro Val Ser Met Ala Lys Thr Ser Val Asp Cys Asn
   705 710 715 720
   Met Tyr Ile Cys Gly Asp Ser Thr Glu Cys Ala Asn Leu Leu Leu Gln
   725 730 735
   Tyr Gly Ser Phe Cys Thr Gln Leu Asn Arg Ala Leu Ser Gly Ile Ala
   740 745 750
   Ala Glu Gln Asp Arg Asn Thr Arg Glu Val Phe Ala Gln Val Lys Gln
   755 760 765
   Met Tyr Lys Thr Pro Thr Leu Lys Tyr Phe Gly Gly Phe Asn Phe Ser
   770 775 780
   Gln Ile Leu Pro Asp Pro Leu Lys Pro Thr Lys Arg Ser Phe Ile Glu
   785 790 795 800
   Asp Leu Leu Phe Asn Lys Val Thr Leu Ala Asp Ala Gly Phe Met Lys
   805 810 815
   Gln Tyr Gly Glu Cys Leu Gly Asp Ile Asn Ala Arg Asp Leu Ile Cys
   820 825 830
   Ala Gln Lys Phe Asn Gly Leu Thr Val Leu Pro Pro Leu Leu Thr Asp
   835 840 845
   Asp Met Ile Ala Ala Tyr Thr Ala Ala Leu Val Ser Gly Thr Ala Thr
   850 855 860
   Ala Gly Trp Thr Phe Gly Ala Gly Ala Ala Leu Gln Ile Pro Phe Ala
   865 870 875 880
   Met Gln Met Ala Tyr Arg Phe Asn Gly Ile Gly Val Thr Gln Asn Val
   885 890 895
   Leu Tyr Glu Asn Gln Lys Gln Ile Ala Asn Gln Phe Asn Lys Ala Ile
   900 905 910
   Ser Gln Ile Gln Glu Ser Leu Thr Thr Thr Ser Thr Ala Leu Gly Lys
   915 920 925
   Leu Gln Asp Val Val Asn Gln Asn Ala Gln Ala Leu Asn Thr Leu Val
   930 935 940
   Lys Gln Leu Ser Ser Asn Phe Gly Ala Ile Ser Ser Val Leu Asn Asp
   945 950 955 960
   Ile Leu Ser Arg Leu Asp Lys Val Glu Ala Glu Val Gln Ile Asp Arg
   965 970 975
   Leu Ile Thr Gly Arg Leu Gln Ser Leu Gln Thr Tyr Val Thr Gln Gln
   980 985 990
   Leu Ile Arg Ala Ala Glu Ile Arg Ala Ser Ala Asn Leu Ala Ala Thr
   995 1000 1005
   Lys Met Ser Glu Cys Val Leu Gly Gln Ser Lys Arg Val Asp Phe
   1010 1015 1020
   Cys Gly Lys Gly Tyr His Leu Met Ser Phe Pro Gln Ala Ala Pro
   1025 1030 1035
   His Gly Val Val Phe Leu His Val Thr Tyr Val Pro Ser Gln Glu
   1040 1045 1050
   Arg Asn Phe Thr Thr Ala Pro Ala Ile Cys His Glu Gly Lys Ala
   1055 1060 1065
   Tyr Phe Pro Arg Glu Gly Val Phe Val Phe Asn Gly Thr Ser Trp
   1070 1075 1080
   Phe Ile Thr Gln Arg Asn Phe Phe Ser Pro Gln Ile Ile Thr Thr
   1085 1090 1095
   Asp Asn Thr Phe Val Ser Gly Asn Cys Asp Val Val Ile Gly Ile
   1100 1105 1110
   Ile Asn Asn Thr Val Tyr Asp Pro Leu Gln Pro Glu Leu Asp Ser
   1115 1120 1125
   Phe Lys Glu Glu Leu Asp Lys Tyr Phe Lys Asn His Thr Ser Pro
   1130 1135 1140
   Asp Val Asp Leu Gly Asp Ile Ser Gly Ile Asn Ala Ser Val Val
           1145                    1150                    1155
   Asn Ile  Gln Lys Glu Ile Asp  Arg Leu Asn Glu Val  Ala Lys Asn
       1160                 1165                 1170
   Leu Asn  Glu Ser Leu Ile Asp  Leu Gln Glu Leu Gly  Lys Tyr Glu
       1175                 1180                 1185
   Gln Tyr  Ile Lys Trp Pro Trp  Tyr Val Trp Leu Gly  Phe Ile Ala
       1190                 1195                 1200
   Gly Leu  Ile Ala Ile Val Met  Val Thr Ile Leu Leu  Cys Cys Met
       1205                 1210                 1215
   Thr Ser  Cys Cys Ser Cys Leu  Lys Gly Ala Cys Ser  Cys Gly Ser
       1220                 1225                 1230
   Cys Cys  Lys Phe Asp Glu Asp  Asp Ser Glu Pro Val  Leu Lys Gly
       1235                 1240                 1245
   Val Lys  Leu His Tyr Thr
       1250